# EUROPEAN PATENT SPECIFICATION

(11) **EP 3 938 781 B1**
(45) Date of publication and mention of the grant of the patent: **10.12.2025**
(21) Application number: 20708525.9
(22) Date of filing: 12.03.2020
(51) Int. Cl.: G01N 33/50, G01N 33/68

(54) **IN VITRO**
IN VITRO ZUM ERHALTEN KLINISCHER DATEN BEI INFLAMMATORISCHEN KRANKHEITEN
PROCEDE IN VITRO POUR L'OBTENTION DE DONNÉEES CLINIQUES DANS DES PATIENS SOUFFRANT DE MALADIES INFLAMMATOIRES

(30) Priority: 13.03.2019 EP 19382184
(43) Date of publication of application: 19.01.2022
(73) Proprietor: Fundación para la Formación e Investigación Sanitarias de la Región De Murcia (FFIS), 30120 El Palmar, Murcia (ES)
(72) Inventor: PELEGRÍN VIVANCOS, Pablo, 30120 El Palmar (Murcia) (ES); MARTINEZ BANACLOCHA, Helios, 30120 (El Palmar) Murcia (ES); MARTÍNEZ GARCÍA, Juan José, 59000 Lille (FR); GARCÍA PALENCIANO, Carlos, 30120 El Palmar (Murcia) (ES)
(74) Representative: Clarke, Modet y Cía., S.L.
(86) International application number: PCT/EP2020/056729
(87) International publication number: WO 2020/182964

(56) References cited:
- WO-A1-2004/002518
- WO-A1-2017/042381
- US-A1- 2012 177 632
- MARTIN J LLEWELYN ET AL: "Sepsis biomarkers in unselected patients on admission to intensive or high-dependency care", CRITICAL CARE, BIOMED CENTRAL LTD., LONDON, GB, vol. 17, no. 2, 26 March 2013 (2013-03-26), pages R60, XP021146286, ISSN: 1364-8535, DOI: 10.1186/CC12588
- SCOTT W CANNA ET AL: "An activating NLRC4 inflammasome mutation causes autoinflammation with recurrent macrophage activation syndrome", NATURE GENETICS., vol. 46, no. 10, 1 October 2014 (2014-10-01), NEW YORK, US, pages 1140 - 1146, XP055694157, ISSN: 1061-4036, DOI: 10.1038/ng.3089

## Description

### FIELD OF THE INVENTION

The present invention refers to the medical field. Particularly, it refers to an *in vitro* method for obtaining clinical data in patients suffering from an inflammatory disease, preferably, for predicting gravity and mortality risk among patients suffering from sepsis or for deciding whether to administer a medical treatment to a patient suffering from an auto inflammatory syndrome.

### STATE OF THE ART

Sepsis is a life-threatening condition that arises when the body's response to infection causes injury to its own tissues and organs.

The response of the immune system during sepsis is a complex dynamic process involving an initial inflammatory response followed by an immune deactivation. Exacerbation of any of these responses compromises the life of septic patients. The initial production of proinflammatory cytokines aims to protect the host from the invading pathogens, but it can also damage non-infected tissues and lead to the dysfunction of different organs and systems. Immunosuppression after the inflammatory response is a compensatory response aimed at protecting the host from the excess production of cytokines and other inflammatory factors; however, in sepsis there is a profound leukocyte deactivation that is related to complications in critically ill patients and which leads to secondary fatal infections and the majority of deaths associated with sepsis. Changes in the metabolism of leukocytes during sepsis are associated with the different immune responses in sepsis, whereby the glycolytic pathway is upregulated during the inflammatory response while defects in both glycolysis and mitochondrial respiration lead to immunosuppression. However, the molecular mechanisms in the host that initiate and regulate immunoparalysis in human sepsis are still poorly understood.

Pattern recognition receptors in innate immune cells are able to recognize microbial- or damage-associated molecules and initiate a proinflammatory response. Inflammasomes are important signaling complexes formed by a subgroup of intracellular pattern recognition receptors that activate caspase-1. Caspase-1 promotes a specific type of cell death called pyroptosis and the release of the proinflammatory cytokines IL-1β, IL-18 and the alarmin high mobility group protein B1 (HMGB1), which are all involved in the inflammatory response of sepsis. A promiscuous type of inflammasome is formed by the nucleotide-binding domain, leucine-rich repeat and pyrin domain-containing protein 3 (NLRP3) that can be activated in response to different microbial- or damage-associated molecules, as elevated extracellular concentrations of the nucleotide adenosine triphosphate (ATP) signaling through the P2X purinoceptor 7 (P2X7). Upon activation, NLRP3 oligomerizes and recruits the apoptotic speck-like protein with a caspase activating domain (ASC) into large oligomers that can be found in biological fluids upon inflammasome activation and pyroptosis execution.

The present invention if focused on assessing the activation of NLRP3 inflammasome by the P2X7 receptor in blood leukocytes from a cohort of clinically relevant intra-abdominal origin septic patients and then carry out a follow-up analysis of the same individuals after sepsis resolution. Based on this assessment, the present invention provides an *in vitro* method for predicting mortality risk among patients suffering from sepsis or for deciding whether to administer a medical treatment to a patient suffering from an autoinflammatory syndrome. Regarding, autoinflammatory diseases, this is a group of genetically diverse but clinically similar disorders characterized by recurrent fever associated with rash, serositis, and musculoskeletal involvement. Although they are considered as rare diseases, the increase in the knowledge about their immunopathogenesis has set the bases for a broad spectrum of diseases not only of immunological nature, but also of metabolic, chronic degenerative and inflammatory nature. Despite the limited clinical and genetic diagnostic, there are no functional or biochemistry diagnostic method for autoinflammatory syndromes. We developed a novel functional diagnostic test for these patients that will help the clinician to quickly tailor the treatment for these syndromes. This method will improve the detection and tailor treatment for autoinflammatory syndromes that could also be expanded to other inflammatory, metabolic and chronic degenerative diseases.

### DESCRIPTION OF THE INVENTION

### Brief description of the invention

The invention is defined by the appended claims.

Systemic infection during sepsis induces an exacerbated inflammatory response that is followed by an immunosuppression of the host and thus compromising the life of critically ill patients. Defects in the metabolism and mitochondrial failure are associated with immunocompromised septic patients. The NLRP3 inflammasome is important for establishing an inflammatory response after activation by the purinergic P2X7 receptor. After studying a cohort of individuals with intra-abdominal origin sepsis, we found that monocytes from these patients presented impaired NLRP3 activation when the P2X7 receptor was stimulated. Furthermore, most of the sepsis-related deaths were among patients whose NLRP3 activation was profoundly altered. In monocytes from septic patients, P2X7 receptor was associated with mitochondrial dysfunction, and activation of the P2X7 receptor resulted in mitochondrial damage, which in turn inhibited the NLRP3 activation mediated by HIF-1a. Mortality increased in a mouse model of sepsis when the P2X7 receptor was activated *in viva.* Thus, the present invention reveals a molecular mechanism initiated by the P2X7 receptor that contributes to NLRP3 impairment during infection.

Such as it is shown in the results provided by the present invention, a Receiver operating characteristic (ROC) analysis showed that the release of IL-1β and the formation of ASC specks from monocytes activated in vitro from septic patients and healthy individuals, as well as the release of HMGB1, IL-6 and TNF-α, were sufficiently significant in the ROC analysis to identify a group of septic patients with impaired NLRP3 inflammasome activation, all with an area under the curve from 0.9 to 1. Particularly, the ROC analysis provided by the present invention for IL-113, ASC-speck formation, HMBG1, IL-6, TNF-α, comparing control vs septic individuals and septic individuals vs those septic individuals with a profound NLRP3 deactivation, reveals that said biomarkers from monocytes stimulated in vitro can be efficiently used according to the present invention for predicting mortality risk among patients suffering from sepsis.

Moreover, according to the present invention, IL-1β, IL-18, ASC-speck formation, HMBG1, IL-6 and/or TNF-α can be efficiently used according to the present invention for deciding whether to administer a medical treatment to a patient suffering from an autoinflammatory syndrome.

Thus, in a preferred embodiment, any of the biomarkers IL-1β, IL-18, ASC-speck formation, HMBG1, IL-6, IL-8 and/or TNF-α can be individually used, after the activation of NLRP3 inflammasome, for predicting mortality risk among patients suffering from sepsis and/or for deciding whether to administer a medical treatment to a patient suffering from an autoinflammatory syndrome. Nevertheless the present invention also comprises the possible use of any combination of at least two, at least three, at least four, at least five or at least six of said biomarkers. In other words, the present invention also refers to a biomarker signature comprising any individual biomarker of the group: IL-1β, ASC-speck formation, HMBG1, IL-6, IL-8, TNF-α, or any combination thereof.

Consequently, the special technical feature which provides unity of invention is the activation of NLRP3 inflammasome. Once the NLRP3 inflammasome is activated, the decreased production of any of the biomarkers IL-1β, IL-18, ASC-speck formation, HMBG1, IL-6, IL-8 and/or TNF-α can be determined in order to implement the present invention.

Particularly, the first embodiment of the present invention refers to an *in vitro* method for obtaining clinical data in patients suffering from an inflammatory disease which comprises determining the concentration level of at least one biomarker selected from the group comprising: IL-1β, IL-18, ASC-specks, HMGB1, IL-6 and/or TNF-α, once the NLRP3 inflammasome has been activated in vitro in blood cells obtained from the patient.

In a preferred embodiment, the present invention refers to an *in vitro* method for predicting mortality risk among patients suffering from sepsis which comprises determining the concentration level of at least one biomarker selected from the group comprising: IL-1β, ASC-specks, HMGB1, IL-6 and/or TNF-α, once the NLRP3 inflammasome has been activated in blood cells obtained from the patient, wherein a variation, preferably a decrease of the concentration level of at least one of said biomarkers with respect to the concentration level determined in control healthy patients, is an indication of mortality risk.

In a preferred embodiment, the present invention refers to an *in vitro* method for deciding whether to administer a medical treatment to a patient suffering from an autoinflammatory syndrome which comprises determining the concentration level of at least one biomarker selected from the group comprising: IL-1β, IL-18, ASC-specks, HMGB1, IL-6 and/or TNF-α, once the NLRP3 inflammasome has been activated in blood cells obtained from the patient, wherein a variation, preferably decrease of the concentration level of at least one of said biomarkers with respect to the concentration level determined in healthy patients, is an indication that a treatment directed to the inhibition of IL-1β, IL-18, ASC-specks, HMGB1, IL-6 and/or TNF-α can be discarded or wherein a variation, preferably an increase of the concentration level of said biomarkers with respect to the concentration level determined in healthy patients, is an indication that a treatment directed to the inhibition of IL-1β, IL-18, ASC-specks, HMGB1, IL-6 and/or TNF-α can be implemented.

In a preferred embodiment, the determination of the concentration level of the biomarkers is carried out in a suitable media comprising NaCl, HEPES, d-glucose, KCI, CaCl₂ and MgCl₂, preferably 147 mM NaCl, 10 mM HEPES, 13 mM d-glucose, 2 mM KCI, 2 mM CaCl₂, and 1mM MgCl₂ and pH 7.4 once the NLRP3 inflammasome has been activated in blood cells obtained from the patient.

In a preferred embodiment, the NLRP3 inflammasome activation is carried out by means of the activation of P2X7 receptor.

In a preferred embodiment, the NLRP3 inflammasome activation is carried out by other means of the activation of P2X7 receptor with LPS and nigericin, uric acid crystals, alum crystals, intracellular lipids or other inflammasomes as NLRC4 or Pyrin inflammasomes are also activated.

In a preferred embodiment, the blood cells in which the NLRP3 inflammasome is activated are leukocytes, preferably monocytes.

In a preferred embodiment, the blood cells in which the NLRP3 inflammasome is activated are peripheral blood mononuclear cells (PBMC) extracted from whole blood samples.

In a preferred embodiment, the above cited method is performed within 5 days from the patient admission.

In a preferred embodiment, the mortality risk is evaluated within a period of 10-90 days.

In a preferred embodiment, the method of the invention comprises the following steps:
a) Adding to the blood samples obtained from the patient a buffer suitable for diluting the blood samples which comprises molecules able to perform the NLRP3 inflammasome activation selected from the list comprising: LPS and ATP, LPS and nigericin, LPS and uric acid crystals, LPS and alum crystals, intracellular LPS delivery or other inflammasomes as NLRC4 or Pyrin inflammasomes,
b) Centrifugating the blood samples,
c) Determining the concentration level of at least one biomarker selected from the group consisting of: IL-1β, IL-18, HMGB1, IL-6 and/or TNF-α, which are comprised in the supernatant, preferably by ELISA or multiplexing,
d) Determining the concentration level of ASC-specks which is comprised in the cellular pellet preferably by flow cytometry.

The second embodiment of the present invention refers to the *in vitro* use of the concentration level of at least one biomarker selected from the group comprising: IL-113, IL-18, ASC-specks, HMGB1, IL-6 and/or TNF-α, once the NLRP3 inflammasome has been activated in blood cells comprised in blood samples obtained from the patient, for obtaining clinical data in patients suffering from an inflammatory disease.

In a preferred embodiment the present invention refers to the *in vitro* use of at least one biomarker selected from the group comprising: IL-113, IL-18, ASC-specks, HMGB1, IL-6 and/or TNF-α, once the NLRP3 inflammasome has been activated in blood cells obtained from the patient, for predicting mortality risk among patients suffering from sepsis or for deciding whether to administer a medical treatment to a patient suffering from an autoinflammatory syndrome.

Related to the present invention is a kit of parts, adapted for performing any of the methods described in the present invention, which comprises at least three components:
a) Buffer suitable for diluting blood samples obtained from the patient which comprises molecules able to perform the NLRP3 inflammasome activation in blood cells.
b) Reagents for determining, preferably by ELISA or multiplexing, the concentration level of at least one biomarker selected from the group consisting of: IL-113, IL-18, HMGB1, IL-6 and/or TNF-α, and
c) Reagents for determining, preferably by flow cytometry, the concentration level of ASC-specks.

In a preferred embodiment, the buffer of the component a) comprises: LPS, ATP, nigericin, uric acid crystals, alum crystals, or other compounds to activate the inflammasomes NLRC4 or Pyrin.

The fourth embodiment of the present invention refer to a method for treating septic patients or patients suffering from an inflammatory disease which initially comprises determining the concentration level of at least one biomarker selected from the group comprising: IL-113, IL-18, ASC-specks, HMGB1, IL-6 and/or TNF-α, once the NLRP3 inflammasome has been activated in blood cells obtained from the patient.

For the purpose of the present invention the following terms are defined:
- The expression "concentration level determined in control healthy patients", refer to a "reference value" of the concentration level of the biomarkers. If the concentration levels of the biomarkers measured in the patients are statistically lower as compared with said "concentration level determined in control healthy patients", which is used as a "reference value", this is an indication of mortality risk or an indication that a treatment directed to the inhibition of the biomarkers can be discarded.
   - A "reference value" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare the biomarker levels (or scores) obtained according to the method of the invention with a defined threshold value. Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0.
- The term "mortality risk" refers to the estimation of the likelihood of in-hospital death for a patient. According to the present invention, mortality risk exists when the concentration levels of the biomarkers are lower, as compared with said "concentration level determined in control healthy subjects" which is used as a "reference value".
- By "comprising" it is meant including, but not limited to, whatever follows the word "comprising". Thus, use of the term "comprising" indicates that the listed elements are required or mandatory, but that other elements are optional and may or may not be present.
- By "consisting of" it is meant "including, and limited to", whatever follows the phrase "consisting of". Thus, the phrase "consisting of" indicates that the listed elements are required or mandatory, and that no other elements may be present.

### Brief description of the figures.

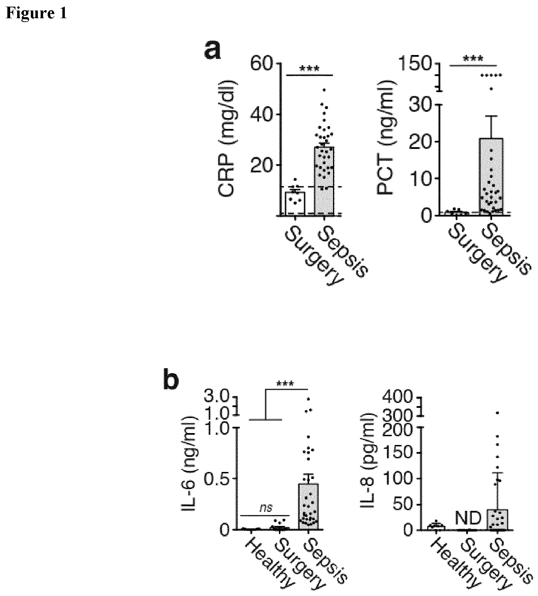

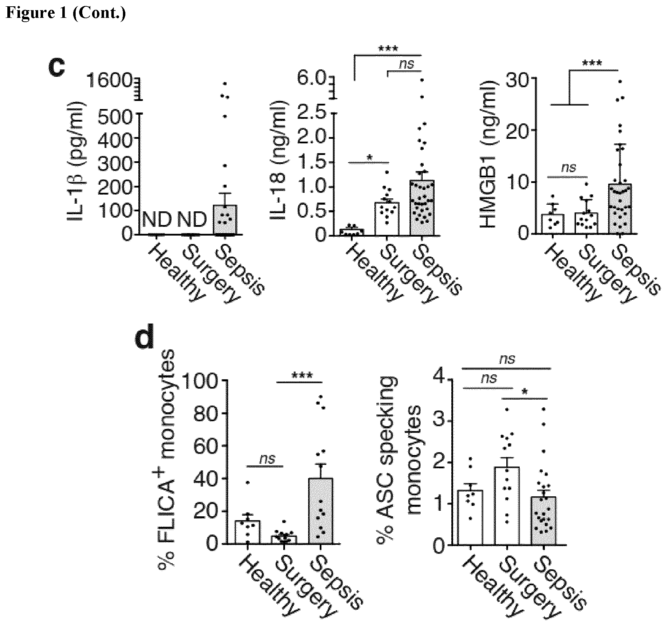

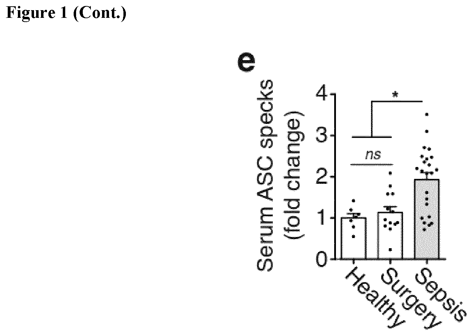

**Figure 1****. Septic patients present elevated inflammasome markers. (a-c)** Plasma concentration of CRP, PCT **(a),** IL-6, IL-8 **(b),** IL-1β, IL-18 and HMGB1 **(c)** in healthy controls, abdominal surgery patients within the first 24 h after surgery and intra-abdominal origin septic patients within the first 24 h of admission to the surgical critical unit; dotted lines in **(a)** represent standard threshold for CRP and PCT in healthy population. **(d)** Percentage of monocytes from healthy controls and septic patient positives for active caspase-1 labelled with FLICA (left) or positives for intracellular ASC specks (right). **(e)** Fold increase of circulating ASC specks in the plasma of surgery controls and septic patients compared with healthy controls. Each dot represents an individual patient; average ± standard error is represented in all panels; *p< 0.05; **p< 0.01; ***p< 0.001; *ns,* no significant difference (p> 0.05); Mann-Whitney test for a; Kruskal-Wallis test for b-e.

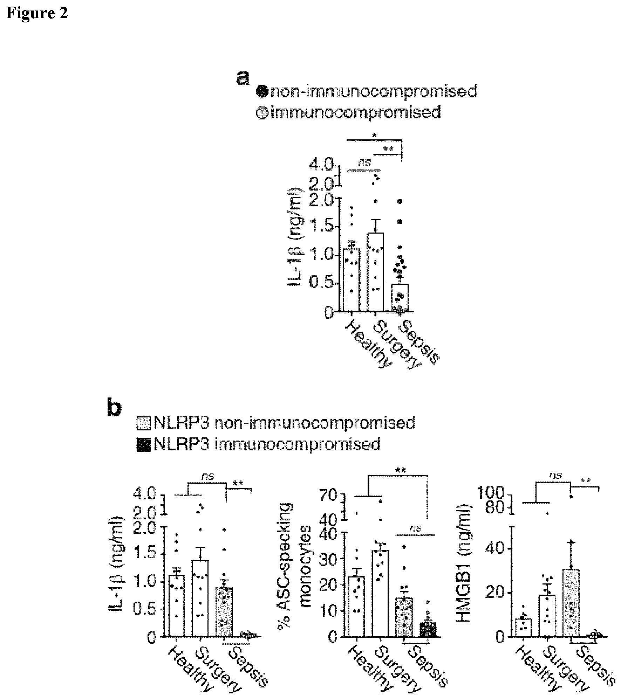

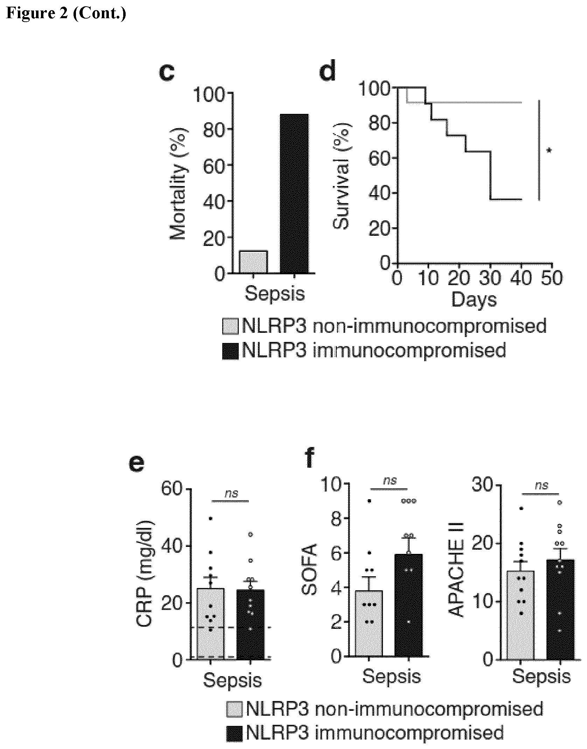

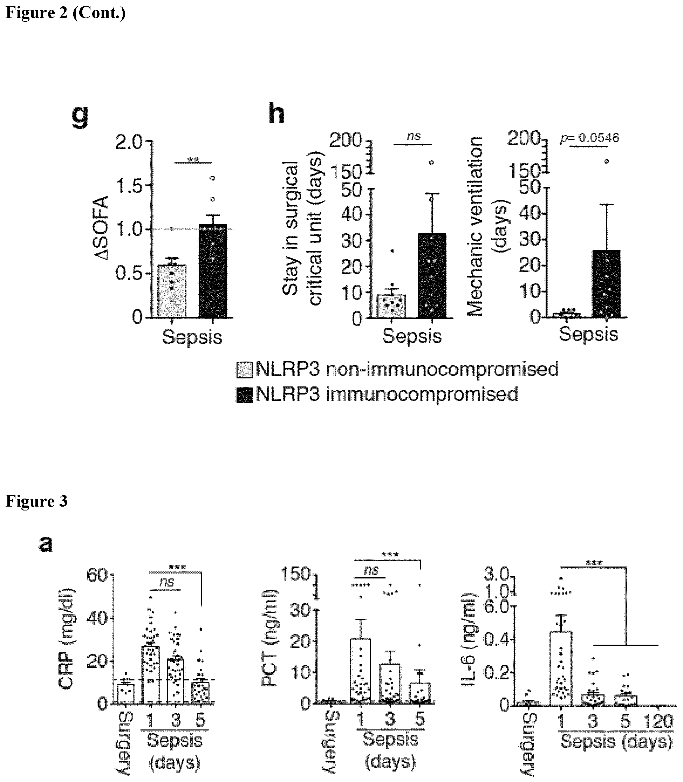

**Figure 2****. Inflammasome activation is compromised in septic patients with high mortality. (a)** ELISA for IL-1β in PBMC supernatants and percentage of monocytes with intracellular ASC specks from septic patients within the first 24 h of admission to the surgical critical unit and control groups after NLRP3 inflammasome activation by LPS (1 µg/ml, 2 h) and ATP (3 mM, 30 min) treatment. There is a group of septic patients that release low or no IL-1β (grey dots). **(b)** ELISA for IL-1β and HMGB1 in PBMC supernatants and percentage of monocytes with intracellular ASC specks from control groups and septic patients treated as in **a,** separating septic patients into two groups: NLRP3 non-immunocompromised (grey bar) and immunocompromised (black bar). **(c)** Percentage of mortality in NLRP3 non-immunocompromised (grey bar) and immunocompromised (black bar) septic patients with regard to the total mortality of septic patients. **(d)** Kaplan-Meier representation of NLRP3 non-immunocompromised (grey line) and immunocompromised (black line) septic patients' survival. **(e)** Concentration of C-reactive protein (CRP) in plasma of NLRP3 non-immunocompromised (grey bar) and immunocompromised (black bar) septic patients at day 1; dotted lines represent threshold concentration of CRP for healthy population. **(f)** SOFA and APACHEII indexes in NLRP3 non-immunocompromised (grey bar) and immunocompromised (black bar) septic patients at day 1. **(g)** ΔSOFA calculated as the variation in SOFA between day 5 and day 1, grey dotted line represents no change with regard to day 1. **(h)** Number of days admitted to the Surgical Critical Unit and number of days of mechanical ventilation for NLRP3 non-immunocompromised (grey bar) and immunocompromised (black bar) septic patients. Each dot represents an individual patient; average ± standard error is represented in panels a,b,e-h; *p< 0.05; **p< 0.01; ***p< 0.001; *ns,* no significant difference (p> 0.05); Kruskal-Wallis test for a-b; Long-rank test for d; and Mann-Whitney test for e-h.

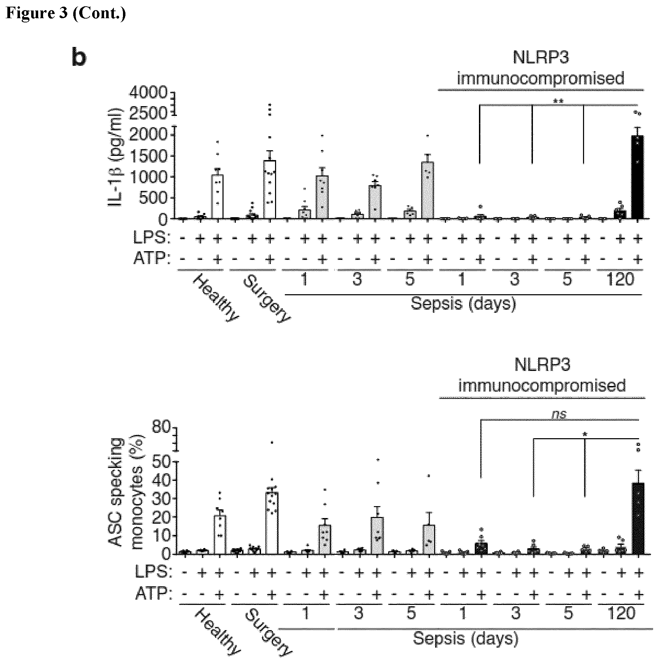

**Figure 3****. NLRP3 immunoparalysis during sepsis is transitory. (a)** Concentrations of C-reactive protein (CRP), procalcitonin (PCT) and IL-6 in plasma of septic patients at days 1, 3 and 5 during sepsis and at day 120 after sepsis recovery; dotted lines represent normal concentration of CRP and PCT. Levels of these markers in septic patients were compared with the abdominal surgery controls at 24 hours after surgery. Control group and septic patients at day 1 correspond to patient data presented in figure 1a and b, and are shown here for comparison. **(b)** ELISA for IL-1β in PBMC supernatants (top) and percentage of monocytes with intracellular ASC specks (bottom) after NLRP3 inflammasome activation by LPS (1 µg/ml, 2h) and ATP (3 mM, 30 min) treatment from control groups and NLRP3 non-immunocompromised (grey) or NLRP3 immunocompromised (black) septic patients at day 1, 3, 5 during sepsis and at day 120 after sepsis recovery. Control groups and septic patients at day 1 correspond to patient data presented in figure 2a and b, and are shown here for comparison; each dot represents an individual patient; average ± standard error is represented in all panels; *p< 0.05; **p< 0.01; ***p< 0.001; *ns,* no significant difference (p> 0.05); Kruskal-Wallis test for a,b.

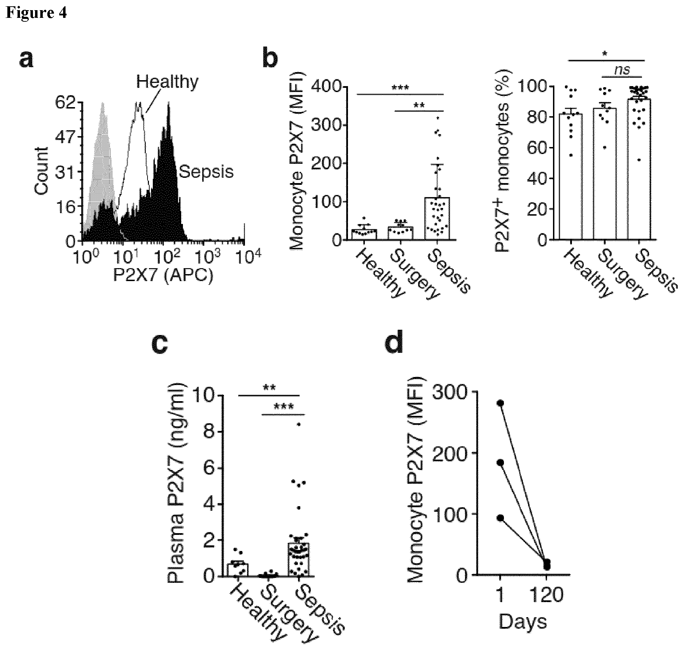

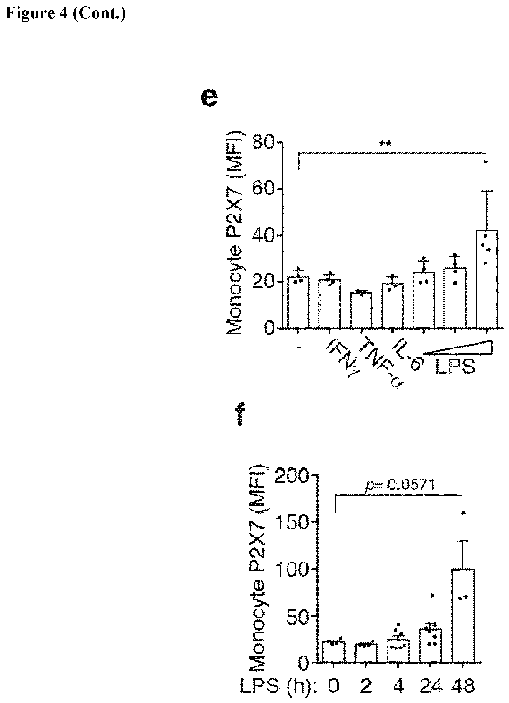

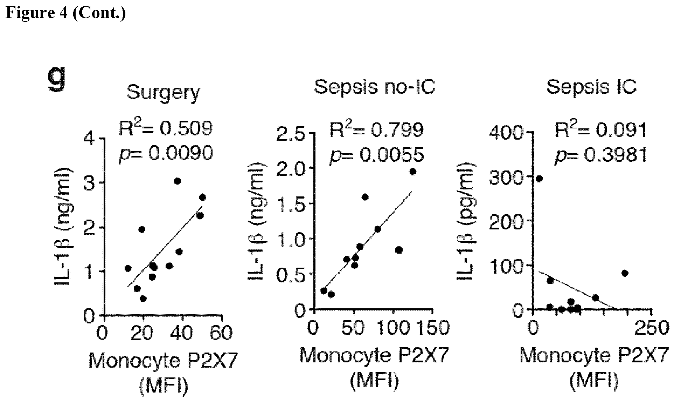

**Figure 4****. P2X7 receptor transiently upregulates in monocytes during sepsis. (a)** Representative histogram plot of surface P2X7 receptor staining in monocytes from healthy (white), septic patient (black) and non-stained monocytes (grey). **(b)** Quantification of P2X7 receptor mean fluorescence intensity (MFI, left) and percentage of positive monocytes for P2X7 receptor (right) in control and septic patients. **(c)** ELISA to quantify the concentration of soluble P2X7 receptor in plasma of control and septic patients. **(d)** Quantification of P2X7 receptor MFI at day 1 during sepsis and day 120 after recovery. **(e)** Quantification of P2X7 receptor MFI in monocytes from healthy donors treated with IFNy, TNF-α, IL-6 (all at 20 ng/ml), or with increasing concentrations of LPS (10, 100, 1000 ng/ml) for 24 h. **(f)** Quantification of P2X7 receptor MFI in monocytes from healthy donors treated with LPS (1 µg/ml) for the indicated times. **(g)** Correlation between the concentration of IL-1β released from PBMCs treated with LPS (1 µg/ml, 2h) and ATP (3 mM, 30 min) and the quantification of P2X7 receptor MFI in monocytes from the indicated control and septic individuals; IC: immunocompromised septic patients. Each dot represents an individual septic patient or healthy donor; average ± standard error is represented in panels b-c,e,f; *p< 0.05; **p< 0.01; ***p< 0.001; *ns,* no significant difference (p> 0.05); Kruskal-Wallis test was used in b; Mann-Whitney test for c,e,f; Pearson correlation was used in g.

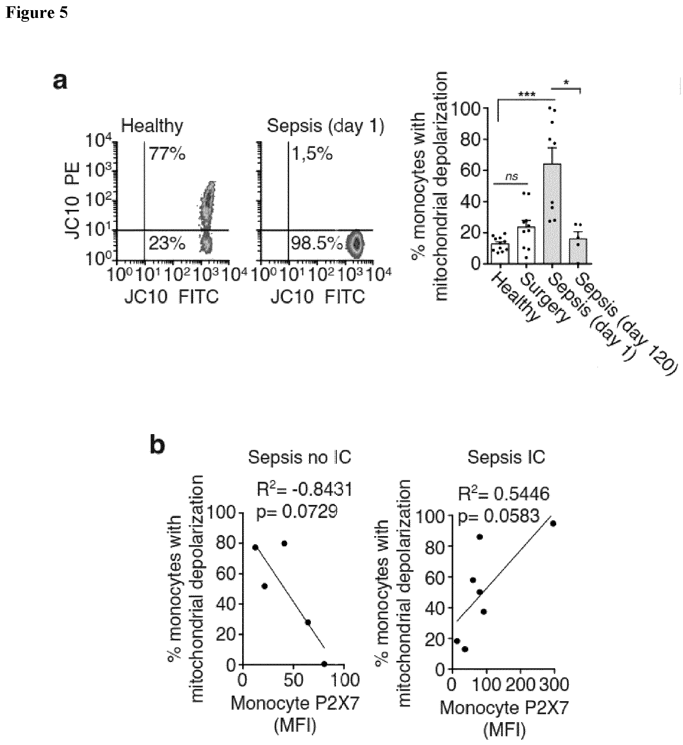

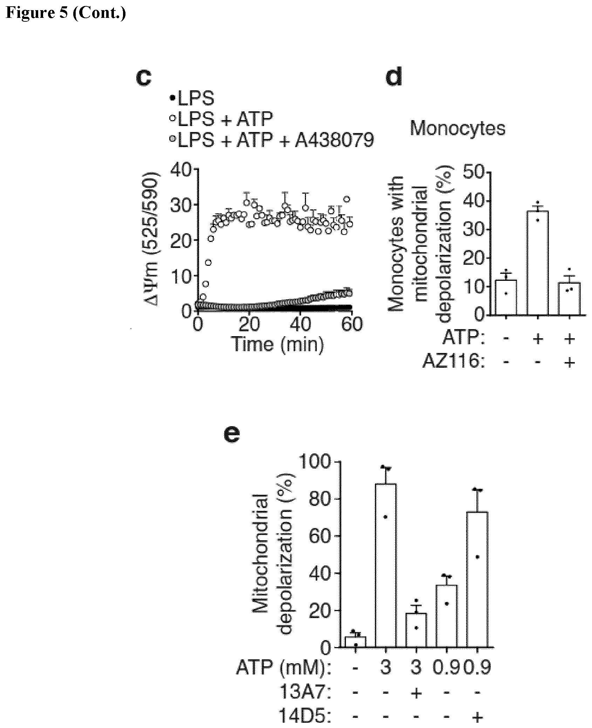

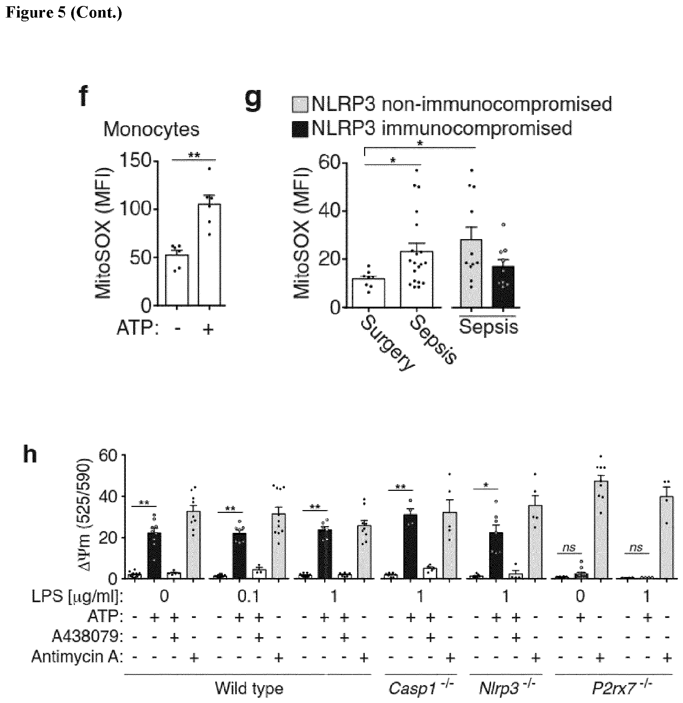

**Figure 5****. P2X7 receptor induces mitochondrial membrane depolarization. (a)** Mitochondrial membrane potential from blood monocytes stained with JC-10 in a control healthy donor (left), in a septic patient at day 1 (middle), and from healthy controls, abdominal surgery and septic patients at day 1 or 120 (right); PE⁻ cells represent the monocytes with mitochondrial depolarization. **(b)** Correlation between the percentage of monocytes with mitochondrial membrane depolarization and the quantification of P2X7 receptor MFI in monocytes from the septic patients; IC: immunocompromised septic patients. **(c)** Kinetics of mitochondrial depolarization after ATP (3 mM) stimulation of LPS-primed BMDMs incubated or not with the P2X7 receptor antagonist A438079 (10 µM). **(d)** Percentage of human monocytes from healthy donors with mitochondrial membrane depolarization after ATP (3 mM, 30 min) stimulation and incubated or not with the P2X7 receptor antagonist AZ11645373 (10 µM). **(e)** Percentage of mouse BMDMs with mitochondrial membrane depolarization after ATP stimulation at the indicated concentrations for 30 min and incubated or not with anti-P2X7 nanobodies (13A7, blocking nanobody; 14D5, potentiating nanobody; each at 200 nM). **(f)** Mitochondrial ROS production from human monocytes isolated from healthy donors after ATP (3 mM, 30 min) stimulation. **(g)** Mitochondrial ROS from human monocytes isolated from surgery controls and septic patients (white bar); right bars separate the group of septic patients into NLRP3 non-immunocompromised (grey bar) and immunocompromised (black bar). **(h)** Mitochondrial membrane depolarization from wild-type or knock-out BMDMs as indicated primed or not with LPS (4 h) and treated for 30 min with ATP (3 mM, black bars), ATP and A438079 (10 µM, white bars), or antimycin A (5 µM, grey bars). Each dot represents an individual patient in a,b,g, or an individual healthy donor in d,f, a single independent experiment in e,h, or average+SEM of two independent experiments in c; average ± standard error is represented in panels a (right),c-h; *p< 0.05; **p< 0.01; ***p< 0.001; *ns,* no significant difference (p> 0.05); Pearson correlation was used in b; Mann-Whitney test was used in f; and Kruskal-Wallis test was used in a,g,h.

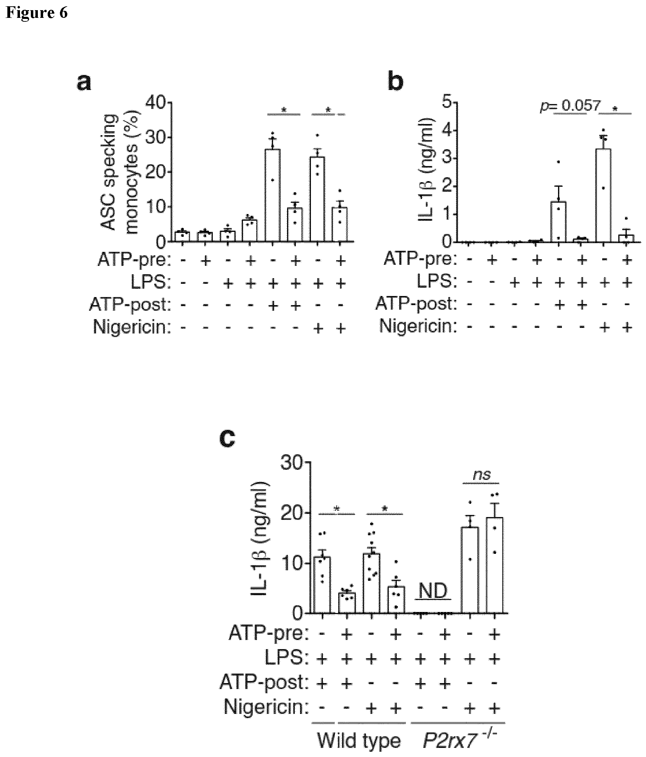

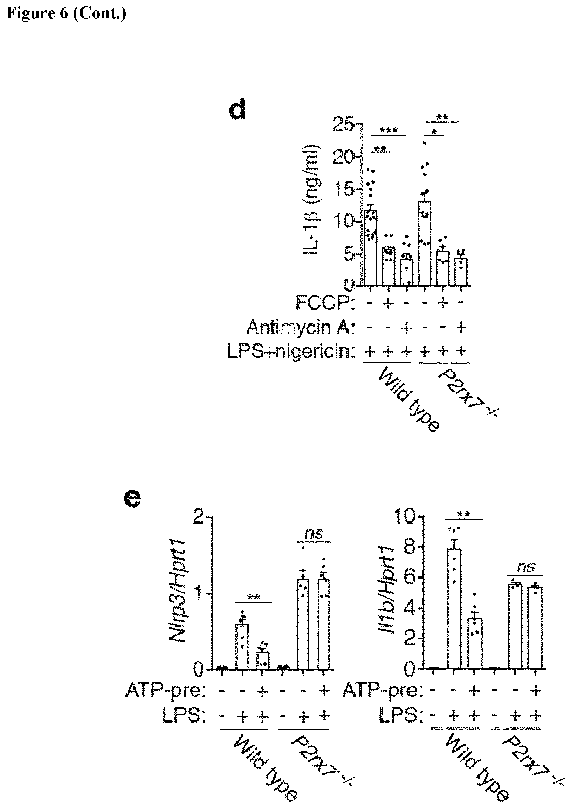

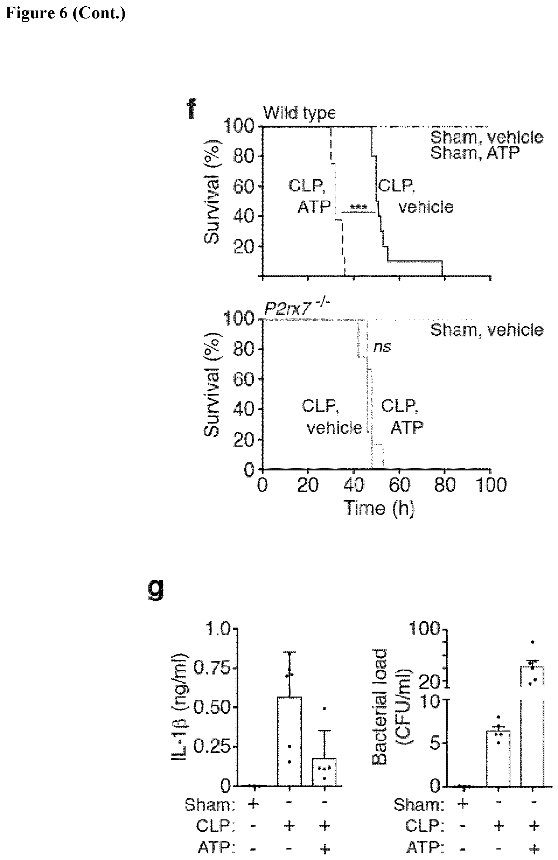

**Figure 6****. P2X7 receptor stimulation induces an impairment of the NLRP3 inflammasome in monocytes. (a-b)** Percentage of monocytes with ASC-specks **(a)** and release of IL-1β from PBMCs **(b)** isolated from healthy donor blood samples treated with ATP (3 mM, 30 min; ATP-pre), then washed and primed with or without LPS (1 µg/ml, 2h) and then treated for 20 min with ATP (3 mM; ATP-post) or nigericin (10 µM) as indicated. **(c)** IL-1β release from wild type or *P2rx7*^{*-*/*-*} BMDMs treated as in **a,** but with 4 h LPS priming and 30 min of ATP or nigericin. **(d)** IL-1β release from wild type or *P2rx7*^{*-*/*-*} BMDMs treated for 30 min with antimycin A (5 µM) or FCCP (1 µM) and then washed and primed with LPS (1 µg/ml, 4h) and then stimulated with nigericin (10 µM, 30 min) as indicated. **(e)** Expression of *Nrlp3* and *II1b* genes analysed by quantitative PCR from wild type or *P2rx7*^{*-*/*-*} BMDMs treated with ATP (3 mM, 30 min; ATP-pre), then washed and primed with or without LPS (100 ng/ml, 4h). **(f)** Kaplan-Meier representation of wild type (top) or *P2rx7*^{*-*/*-*} (bottom) mice survival after sham operation (dotted line), or CLP operation (continuous line); some mice groups were i.p. injected with ATP (0.5 mg/g) 30 min before operation (dashed lines). Wild type CLP *n=* 10, CLP+ATP *n*= 8, sham *n*= 4, sham+ATP *n*=4; *P2rx7*^{*-*/*-*} CLP *n*= 4, CLP+ATP *n*= 6, sham *n*= 3. **(g)** IL-1β from peritoneal lavage (left) or bacterial load in blood (right) from wild type sham, CLP or CLP+ATP mice after 24 h. Each dot represents a single independent experiment (c,d), a sample from an individual healthy donor (a,b,e) or a single mouse (g); average ± standard error is represented in panels a-e,g; *p< 0.05; **p< 0.01; *ns,* no significant difference (p> 0.05); Mann-Whitney test was used for a,b,d,e; Kruskal-Wallis test was used for c; Long-rank test for f.

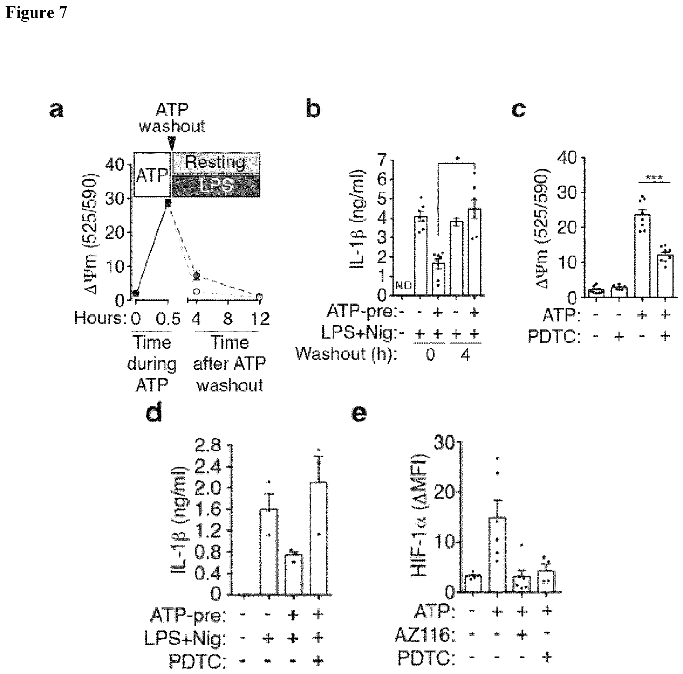

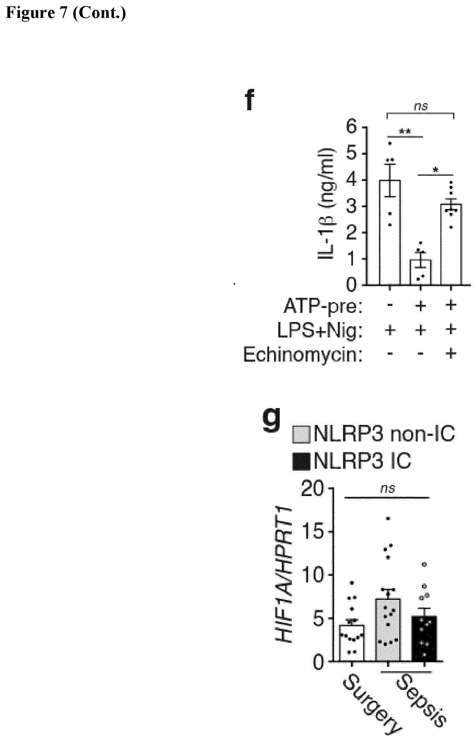

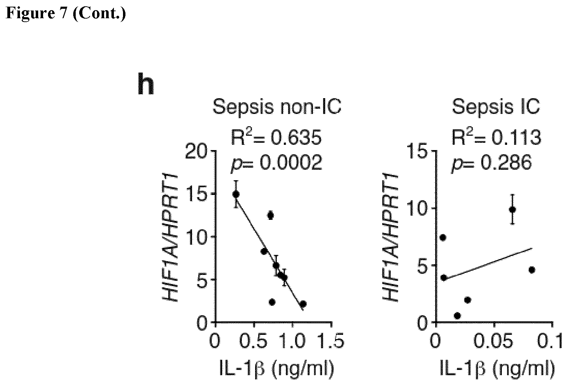

**Figure 7****. Mitochondrial dysfunction mediates P2X7 receptor induced NLRP3 inflammasome impairment. (a)** Mitochondrial membrane depolarization in BMDMs treated with ATP (3 mM, 30 min), then washed out and incubated for the indicated times in the absence or presence of LPS (1 µg/ml) as indicated. **(b)** IL-1β release from wild-type BMDMs treated as in **a,** but after LPS priming cells were stimulated with nigericin (10 µM, 30 min). **(c)** Mitochondrial membrane depolarization in BMDMs treated with ATP (3 mM, 30 min) in the presence or absence pyrrolidine dithiocarbamate (PDTC, 40 µM). (d) IL-1β release from PBMC supernatants isolated from healthy donor blood samples treated with ATP (1 mM, 30 min; ATP-pre) in the presence or absence of PDTC (10 µM), then washed and primed with or without LPS (1 µg/ml, 4h) and then stimulated with nigericin (10 µM, 30 min). **(e)** Representative histogram plot of HIF-1α staining (left) or quantification of HIF-1α mean intensity fluorescence increase (ΔMFI, right) in monocytes isolated from healthy donor blood samples and treated or not with ATP (1 mM, 30 min) in the presence or absence of AZ11645373 (10 µM) or PDTC (10 µM), and then washed and cultured for 2h; non-stained monocytes (light grey, left). **(f)** IL-1β release from BMDM supernatants treated with ATP (3 mM, 30 min; ATP-pre) in the presence or absence of echinomycin (5 nM), then washed and primed with or without LPS (1 µg/ml, 4h) and then stimulated with nigericin (10 µM, 30 min). **(g)** Expression of *HIF1A* gene analysed by quantitative PCR from control surgery group and septic patients PBMCs; septic patients are separated into NLRP3 non-immunocompromised (grey bar) and immunocompromised (black bar). **(h)** Correlation between the expression of *HIF1A* in PBMCs and the concentration of IL-1β released from PBMCs treated with LPS (1 µg/ml, 2h) and ATP (3 mM, 30 min) from septic patients; IC: immunocompromised septic patients. Each dot represents a single independent experiment or a sample from an individual healthy donor or septic patient; average ± standard error is represented in panels a-g; *p< 0.05; **p< 0.01; ***p< 0.001; *ns,* no significant difference (p> 0.05); Kruskal-Wallis test was used for b,c,f; Pearson correlation was used in h.

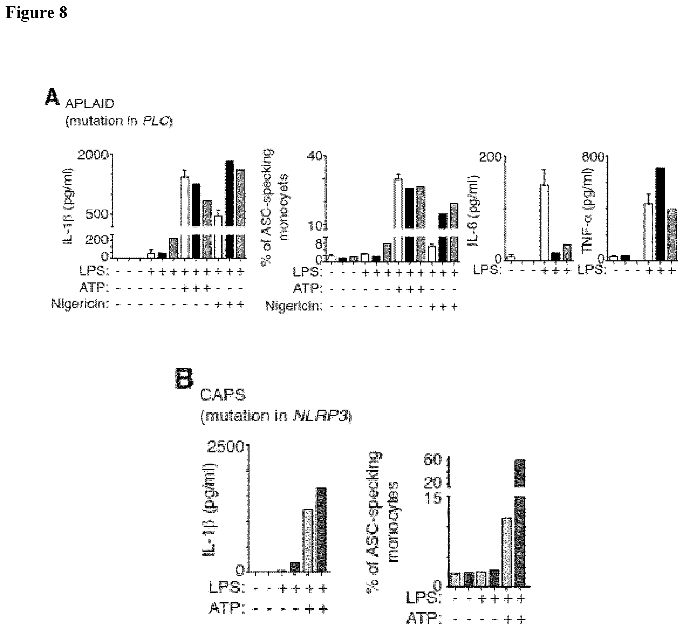

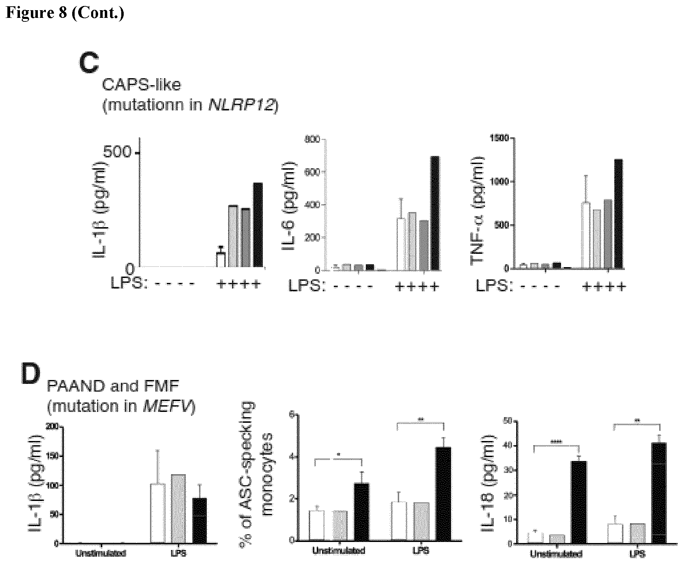

**Figure 8****. Development of a functional diagnostic tool for patients with autoinflammatory syndromes.** This figure shows different patients with different mutations in different genes and different autoinflammatory syndromes, release different patterns of cytokines. In panel **a)** two APLAID syndromes (black and grey) with mutations in the gen PLC produce normal levels of inflammasome activation (ASC specks) and IL-1β and TNFα production. However, a clear deficiency is found in IL-6 production. In a CAPS syndrome (dark grey, panel **b),** there is an elevated production of IL-1β just after LPS stimulation, that is also increased after LPS+ATP stimulation. This is further enhanced when ASC-specks are measured. In panel **c),** a patient with mutations in NLRP12 (CAPS-like, in black) produce more IL-6 than PBMCs from healthy individuals (white) or CAPS patients with mutations in NLRP3 (grey). In panel **d),** PAAND syndrome PBMCs (black) produce high levels of IL-18 and inflammasome activation (ASC-specks) when stimulated with LPS, when compared with FMF patients (grey) or healthy donors (white).

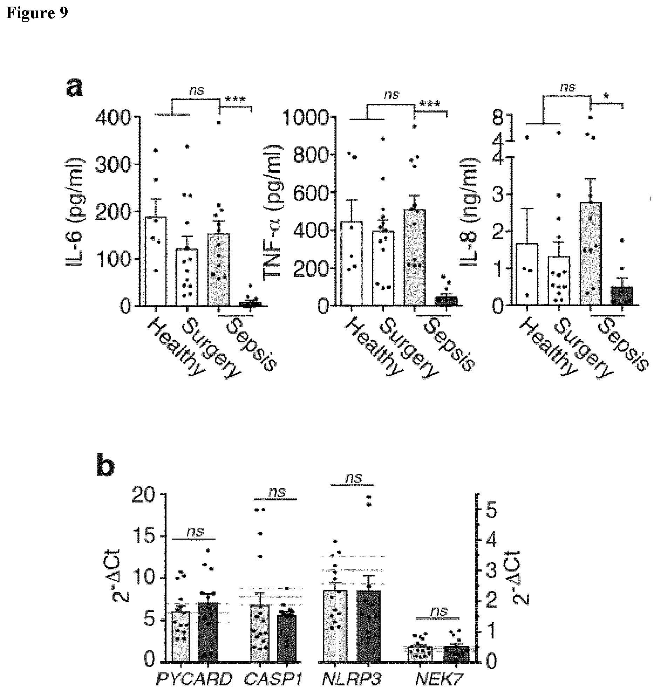

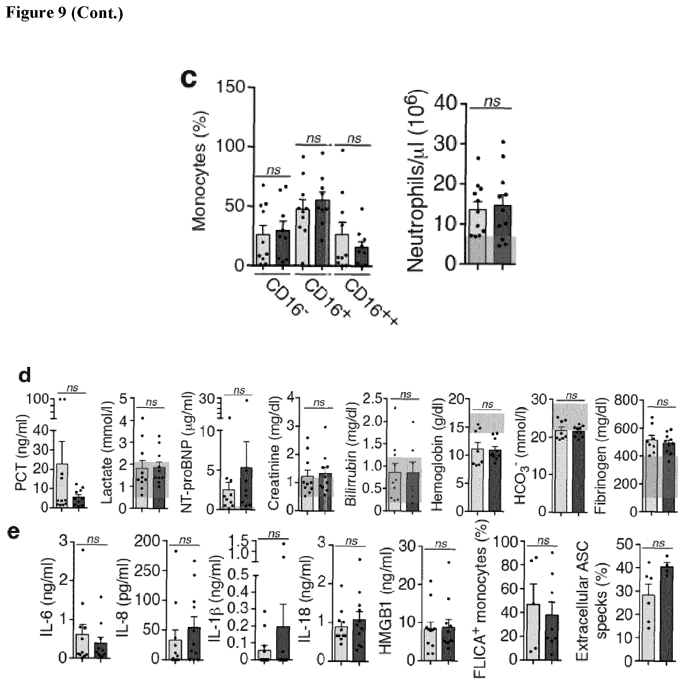

**Figure 9****. Inflammatory and biochemical determinations in septic patients. (a)** Multiplexing for IL-6, TNF-α and IL-8 cytokines in PBMC supernatants from septic patients stratified as in **Figure 2b** and control groups after LPS (1 µg/ml, 2h) treatment. **(b)** Expression of *PYCARD* (ASC*)*, *CASP1, NLRP3* and *NEK7* genes analysed by quantitative PCR from septic patients' PBMCs at day 1; dark grey represents septic patients with a profound deactivation of the NLRP3 inflammasome; the average expression for surgery control group is represented by a continuous grey line for each gene; the s.e.m. for the surgery control group is represented by a dotted grey line for each gene. **(c)** Percentage of CD16 populations on CD14⁺ monocytes (left) and number of circulating neutrophils (right) from septic patients at day 1; dark grey represents septic patients with a profound deactivation of the NLRP3 inflammasome. **(d)** Different clinical markers in the blood from septic patients at day 1; dark grey represents septic patients with a profound deactivation of the NLRP3 inflammasome; grey shadow on graphs indicates the normal range for each parameter analysed. **(e)** Cytokines measured from the plasma (IL-6, IL-8, IL-1β, IL-18 and HMGB1), percentage of monocytes with active caspase-1 (FLICA⁺ monocytes) and presence of circulating ASC specks on plasma from septic patients at day 1; dark grey represents septic patients with a profound deactivation of the NLRP3 inflammasome. Each dot represents an individual patient; *ns,* no significant difference (p> 0.05); Mann-Whitney test for b-e.

### Detailed description of the invention.

### Example 1. MATERIAL AND METHODS.

### Example 1.1. Human clinical samples.

The clinical ethics committee of the Clinical University Hospital *Virgen de la Arrixaca* (Murcia, Spain) approved this study and its procedures (reference number PI13/00174). The samples and data from patients included in this study were provided by the *Biobanco en Red de la Regi6n de Murcia* (PT13/0010/0018), which is integrated into the Spanish National Biobanks Network (B.000859). All study procedures were conducted in accordance with the declaration of Helsinki. Whole peripheral blood samples were collected after receiving written informed consent from intraabdominal septic patients (*n*= 35, **Table 1** showing demographics and clinical characteristics of enrolled patients with intra-abdominal origin sepsis and control groups) at the Surgical Critical Unit from the Clinical University Hospital *Virgen de la Arrixaca* (Murcia, Spain) after 1, 3, 5 and 120 days of sepsis development, day 1 being the blood sample obtained within 24 hours of the diagnosis of sepsis.

Acute physiology and chronic health evaluation II (APACHE II) and sequential organ failure assessment (SOFA), different clinical, microbiological, hemodynamic and biochemical determinations were routinely evaluated in all septic patients at different days by the Clinical University Hospital *Virgen de la Arrixaca* Surgical Critical Unit, Clinical Analysis and Microbiology Units. All the septic patients included in this study met the definition for severe sepsis or septic shock that was valid at the time of sample and data collection. The inclusion criteria for septic patients were patients diagnosed with intra-abdominal origin sepsis confirmed by exploratory laparotomy, with at least two diagnostic criteria for sepsis (fever or hypothermia; heart rate greater than 90 beats per minute; tachypnea, leukocytosis or leukopenia) and multiple organ dysfunction defined as physiological dysfunction in two or more organs or organ systems. We excluded patients who were immunocompromised or presented immunodeficiency (including antineoplastic treatments during the month previous to the septic episode). We also excluded terminal oncologic and hematologic neoplastic patients, as well as patients that had a delay of >24 h from intra-abdominal sepsis diagnosis to surgery, patients who spent less than 24 h in the Surgical Critical Unit, those whose infection was not cleared by the surgery and patients who presented another septic focus different from the abdominal focus. We also analyzed whole peripheral blood samples from healthy volunteers (*n*= 11) and abdominal surgery patients who did not developed sepsis (*n*= 14, **Table 1).**

**Table 1**

| | **Healthy controls** | **Abdominal surgery controls** | **Intra-abdominal origin septic patients** |
|---|---|---|---|
| N | 11 | 14 | 35 |
| **Age,** mean (range) ± SD | 67.45 (49-79) ±10.68 | 66,29 (38-94) ± 16,03 | 69.33 (43-91) ±13.34 |
| *p* value *vs* septic group | *p*> 0.05*^{ns}* | *p*> 0.05*^{ns}* | |

| **Gender,** N (%) | | | |
|---|---|---|---|
| Male | 5 (45%) | 8 (57%) | 21 (60%) |
| Female | 6 (55%) | 6 (43%) | 14 (40%) |
| *p* value *vs* septic group | *p*> 0.05*^{ns}* | *p*> 0.05*^{ns}* | |

| **Clinical data** (*only for septic patients*) | | | |
|---|---|---|---|
| **Anatomical location of the initial focus,** N (%) | | | |
| *Stomach* | | | 3 (8.1%) |
| *Duodenum* | | | 1 (2.7%) |
| *Gallbladder* | | | 5 (13.5%) |
| *Liver* | | | 1 (2.7%) |
| *Small intestine* | | | 6 (16.2%) |
| *Cecum* | | | 1 (2.7%) |
| *Colon* | | | 18 (48.7%) |
| *Sigmoid colon* | | | 1 (2.7 %) |
| *Abdominal, unidentified* | | | 1 (2.7%) |
| **APACHEII score at admission** | | | 18.65 (5-52) ± 7.75 |
| mean (range) ± SD | | | |
| **SOFA score at admission** | | | 6.4 (2-12) ± 2.83 |
| mean (range) ± SD | | | |
| **Days in Surgical Critical Unit** | | | 18.33 (2-167) ± 28.47 |
| mean (range) ± SD | | | |
| **Days of mechanical ventilation** | | | 12.75 (0-167) ± 31.31 |
| mean (range) ± SD | | | |
| **Mortality** N (%) | | | 12 (34.3%) |

| | | | |
|---|---|---|---|
| *SD, standard deviation; ns, no significant difference (p> 0.05); Chi-square (χ²) test was used, except for age, where a one-way ANOVA test was used.* | | | |

### Example 1.2. Cecal ligation and puncture model.

The University of Murcia Animal Research Ethical Committee approved animal procedures (ref. 5/2014) and then the Animal Health Service of the General Directorate of Fishing and Farming of the Council of Murcia (*Servicio de Sanidad Animal, Direcci6n General de Ganaderia y Pesca, Consejería de Agricultura y Agua Regi6n de Murcia*) approved animal procedures with ref. A1320140201. Cecal ligation and puncture (CLP)-induced sepsis was performed in C57BL/6 (WT, wild-type) and P2X7R-deficient (P*2rx7*^{-/-}) mice in C57BL/6 background. 30 min before CLP, a group of mice received an intraperitoneal injection of ATP (0.5 mg/g) or saline vehicle. Laparotomy was performed to isolate the cecum of mice anesthetized with isoflurane. Approximately 2/3 of the cecum was ligated with a 6-0 silk suture and punctured twice through-and-through with a 21 gauge needle. The abdominal wall and incision were then closed with 6-0 silk suture. Sham operated animals underwent laparotomy without ligation or puncture of the cecum. Buprenorphine (0.3 mg/kg) was administered intraperitoneally at the time of surgery and mice were monitored continuously until recovery from anesthesia. For sample collection, 24 h after the procedure, animals were euthanized with CO₂ inhalation and peritoneal lavages were performed with 4 ml of sterile saline and then blood was collected from the thoracic aorta. Serum and centrifuged (cell free) peritoneal lavages were stored at -80°C until further analysis. Serum was diluted serially in sterile physiologic saline and plated and cultured on agar plates at 37°C for 24 h. Then the number of bacterial colonies was counted and expressed as CFU/ml of serum.

### Example 1.3. Cells and treatments.

Human peripheral blood mononuclear cells (PBMCs) were isolated from blood within one hour after extraction using Ficoll histopaque 1077 (Sigma-Aldrich). BMDM were obtained from wild-type, *Nlrp3*^{*-*/}*⁻, Casp1*^{*-*/*-*} and *P2rx7*^{*-*/-} mice as previously described. THP-1 cells were maintained in RPMI1640 media with 10% FCS and 2 mM GlutaMAX (Invitrogen). Cells were treated with ATP (Sigma-Aldrich), antimycin A (Sigma-Aldrich) or FCCP (Sigma-Aldrich) in the presence or absence of PDTC (Sigma-Aldrich) or echinomycin (Sigma-Aldrich) in E-total buffer (147 mM NaCl, 10 mM HEPES, 13 mM glucose, 2 mM CaCl₂, 1 mM MgCl₂, and 2 mM KCI) and then washed and stimulated with *E*. *coli* LPS O55:B5 in their respective complete media. In some experiments, PBMCs were activated with recombinant human IL-6, TNF-α or IFNγ (PeproTech). After LPS treatment, cells were incubated with P2X7 modulating nanobodies, the specific P2X7 receptor antagonist A438079 or AZ11645373 (Tocris), and then subsequently stimulated with ATP or nigericin (Sigma-Aldrich) in E-total buffer. Times and concentrations for the reagents used are specified in the figure legends. 23 out of the 35 septic patients included in this study (65.7%) were able to provide enough blood samples for *in vitro* PBMC stimulation.

### Example 1.4. ELISA and multiplexing assay.

Cytokines and soluble P2X7 in plasma were measured by ELISAs from eBioscience, IBL International, R&D Systems, Cusabio (for soluble P2X7) and MBL following the manufacturers' indications and read in a Synergy Mx (BioTek) plate reader. Multiplexing was performed using the Cytometric Bead Array from Becton Dickinson Biosciences following the manufacturer indications, and were analysed in a BD FACS Canto.

### Example 1.5. Flow cytometry.

Intracellular ASC-speck formation was evaluated by the Time of Flight Inflammasome Evaluation in CD14⁺ monocytes using a polyclonal unconjugated rabbit anti-ASC (N-15)-R antibody (SantaCruz Biotechnology) and a secondary monoclonal donkey anti-rabbit antibody Alexa Fluor-488 (Thermofisher Scientific), both at 1:1000 dilution. HIF-1α expression was detected in CD14⁺ monocytes using Alexa Fluor-647 mouse anti-human HIF-1α (BD Biosciences, Clone 54) at 1:1000 dilution. Monocytes were determined from PBMCs were determined by CD3⁻ CD14⁺ selection and P2X7 receptor surface expression was determined in CD16^{-/+/++} monocytes using the monoclonal anti-P2X7 L4 clone conjugated with APC⁶². Active caspase-1 was measured in monocytes using the specific fluorescent probe FLICA-660 Caspase-1 Assay Kit (Immunochemistry Technologies) following the manufacturer's instructions. Production of ROS was measured in monocytes using the red mitochondrial superoxide indicator MitoSOX (Invitrogen) following the manufacturer's instructions. The detection of extracellular particles of ASC was performed on 0.5 ml of human plasma. For the determination of ASC specks, HIF-1α expression and FLICA, monocytes were selected using anti-CD14 PE (clone 61D3, Tonbo biosciencies) or anti-CD14 APC-H7 (clone MφP9, BD Biosciences). For MitoSOX determination monocytes were selected using anti-CD33 APC-vio770 (clone REA775, Miltenyi). Samples were analysed by flow cytometry using FACS Canto (BD Biosciences) and the FCS express software (De Novo Software).

### Example 1.6. Mitochondrial membrane potential.

Mitochondrial membrane potential was measured using the JC-10 dye (Abcam) in BMDMs with a Sinergy Mx plate reader (BioTek) or by flow cytometry using the BD FACS Celesta flow cytometer (BD Biosciences), or in human monocytes (CD14⁺ labelled with anti-CD14 APC-H7, clone MφP9, BD Biosciences) using FACS Canto (BD Biosciences), following the indications of manufacturer.

### Example 1.7. LDH assay.

Presence of LDH in cell-free supernatants was measured using the Cytotoxicity Detection kit (Roche) following the manufacturer's instructions.

### Example 1.8. Quantitative reverse transcriptase-PCR analysis.

Detailed methods used for qRT-PCR have been described in the prior art. Relative gene expression levels were calculated using the 2^{-ΔCt} method normalizing to *Hprt1* expression as endogenous control.

### Example 1.9. Statistical analysis.

Statistics were calculated with Prism software (GraphPad Software Inc.). Normality of the samples was determined with D'Agostino and Pearson omnibus K2 normality test and samples did not follow a Gaussian distribution. Outliers from data sets were identified by the ROUT method with Q=1%. Non-parametric Mann-Whitney test was used to analyse differences between two non-paired groups, Wilcoxon test was used to compare two paired groups, and Kruskal-Wallis test was used to analyse differences among three or more groups. The χ² test was used to determine whether there was a significant difference between different clinical variables among groups of septic patients. Kaplan-Meier was used to estimate the survival of septic patients and the log-rank test was used to compare the survival distributions of samples.

### Example 2. RESULTS.

### Example 2.1. Inflammasome markers are elevated in sepsis.

We analyzed a cohort of intra-abdominal origin septic patients (*n*= 35, **Table 1)** who presented elevated levels of C-reactive protein (CRP) and procalcitonin (PCT) in their plasma 24 hours after sepsis initiation when compared to a control group of abdominal surgery patients that had not developed sepsis **(****Figure 1a****).** Septic patients presented an average acute physiology and chronic health evaluation (APACHE II) of 18.6 ± 7.7 (range 5 to 52) and a sequential organ failure assessment (SOFA) of 6.4 ± 2.8 (range 2 to 12), with dysfunction of one or more vital organs together with an increase in neutrophils, lactate, NT-proBNP, creatinine, bilirubin and fibrinogen **(Table 1** and **Table 2).** Particularly, **Table 2** shows demographic and clinical data of septic patients.

**Table 2**

| | **Septic patients** | **Septic patients with profound NLRP3 deactivation** |
|---|---|---|
| **N** | 35 | 11 |
| **Age** | | |
| Mean (range) ± SD | 69.33 (43-91) ± 13.34 | 74.64 (53-90) ± 11.64 |
| *p* value against septic group | | *p*> 0.05*^{ns}* |

| **Gender,** N (%) | | |
|---|---|---|
| Male | 21 (60) | 6 (54.5) |
| Female | 14 (40) | 5 (45.5) |
| *p* value against septic group | | *p*> 0.05*^{ns}* |

| **Oncologic patients** | | |
|---|---|---|
| N (%) | 11 (31.4) | 6 (54.5) |
| *p* value against septic group | | *p*> 0.05*^{ns}* |

| **Organ dysfunction,** N (%) | | |
|---|---|---|
| Renal | 17 (48.6) | 6 (54.5) |
| Respiratory | 24 (68.6) | 9 (81.8) |
| Cardiovascular | 26 (74.3) | 10 (90.9) |
| Hepatic | 16 (45.7) | 4 (36.4) |
| Hematological | 5 (14.3) | 0 (0) |
| *p* value against septic group | | *p*> 0.05*^{ns}* |

| **Infection acquired,** N (%) | | |
|---|---|---|
| Nosocomial | 19 (54.3) | 7 (63.6) |
| Community | 16 (45.7) | 4 (36.4) |
| *p* value against septic group | | *p*> 0.05*^{ns}* |

| **Mortality** | | |
|---|---|---|
| N (%) | 12 (34.3) | 7 (63.6) |

| **Mortality cause,** N (%) | | |
|---|---|---|
| Initial multi-organic failure* | 6 (50) | 3 (42.8) |
| Secondary complications** | 6 (50) | 4 (57.2) |

| | | |
|---|---|---|
| *SD, standard deviation; ns, no significant difference (p> 0.05) with Chi-square (χ²) test. *Mortality due to the initial organ dysfunction without clear recuperation after initial admission.* ***Recuperation of initial organ dysfunction after admission, mortality due to acquisition of new infectious processes that could result in another multiple organ dysfunction.* | | |

In contrast, the amounts of hemoglobin and bicarbonate were lower than the standards found in healthy individuals. As expected, IL-6 and IL-8 were also higher in the plasma of septic patients when compared to control surgery and healthy groups **(****Figure 1b****).** However, no IL-12, IL-4, IFNy, IL-2 and TNF-α were found in the plasma of septic patients with the exception of three septic individuals who presented measurable concentrations of TNF-α (see **Table 3** which shows plasma cytokine levels (pg/ml) for septic individuals included in this study (ND: not determined).

**Table 3**

| **Individual** | **IL-1β** | **IL-2** | **IL-4** | **IL-6** | **IL-8** | **IL-12** | **IL-18** | **IFNγ** | **TNF-α** | **HMGB1** |
|---|---|---|---|---|---|---|---|---|---|---|
| 1 | 0 | 0 | 0 | 176.59 | 0 | 0 | 2193.11 | 0 | 0 | 29.32 |
| 2 | 115.88 | 0 | 0 | 238.33 | 22.05 | 0 | 772.39 | 0 | 139.25 | ND |
| 3 | 0 | 0 | 0 | 64.24 | 0 | 0 | 999.36 | 0 | 0 | 16.43 |
| 4 | 51.13 | 0 | 0 | 345.52 | 0 | 0 | 1065.07 | 0 | 600.94 | 26.22 |
| 5 | 0 | 0 | 0 | 158.36 | 0 | 0 | 418.82 | 0 | 0 | 25.78 |
| 6 | 0 | 0 | 0 | 88.66 | 0 | 0 | 661.32 | 0 | 0 | 20.89 |
| 7 | 0 | 0 | 0 | 53.73 | 0 | 0 | 1946.86 | 0 | 0 | 8.72 |
| 8 | 0 | 0 | 0 | 71.46 | 0 | 0 | 649.54 | 0 | 0 | 0 |
| 9 | 0 | 0 | 0 | 98.55 | 0 | 0 | 378.64 | 0 | 0 | 4.80 |
| 10 | 0 | 0 | 0 | 105.67 | 0 | 0 | 971.86 | 0 | 0 | 0 |
| 11 | 0 | 0 | 0 | 68.99 | 0 | 0 | 2288.46 | 0 | 96.20 | 13.34 |
| 12 | 81.43 | 0 | 0 | 311.52 | 0 | 0 | 271.86 | 0 | 0 | 1.33 |
| 13 | 0 | 0 | 0 | 228.74 | 0 | 0 | 336.14 | 0 | 0 | 5.13 |
| 14 | 0 | 0 | 0 | 113.33 | 0 | 0 | 723.46 | 0 | 0 | 9.80 |
| 15 | 0 | 0 | 0 | 80.08 | 0 | 0 | 439.00 | 0 | 0 | 7.90 |
| 16 | 796.60 | 0 | 0 | 763.26 | 0 | 0 | 516.87 | 0 | 0 | 6.38 |
| 17 | 0 | 0 | 0 | 754.84 | 34.05 | 0 | 1899.69 | 0 | 0 | 19.77 |
| 18 | 0 | 0 | 0 | 266.07 | 88.54 | 0 | 1318.44 | 0 | 0 | 16.21 |
| 19 | 0 | 713.82 | 0 | 2790.15 | 182.46 | 0 | 809.06 | 0 | 0 | 1.84 |
| 20 | 81.43 | 0 | 0 | 1416.87 | 0 | 0 | 466.87 | 0 | 0 | 8.01 |
| 21 | 0 | 0 | 0 | 691.51 | 0 | 0 | 663.75 | 0 | 0 | 5.26 |
| 22 | 0 | 0 | 0 | ND | 317.91 | 0 | 721.56 | 0 | 0 | 10.35 |
| 23 | 0 | 0 | 0 | 798.16 | 11.02 | 0 | 571.56 | 0 | 0 | 1.87 |
| 24 | 0 | 0 | 0 | 103.35 | 0 | 0 | 5584.06 | 0 | 0 | 10.31 |
| 25 | 488.95 | ND | ND | 137.62 | 121.92 | ND | 3254.36 | ND | ND | 8.16 |
| 26 | 1365.63 | ND | ND | 1583.34 | 142.22 | ND | 294.68 | ND | ND | 5.14 |
| 27 | 0 | ND | ND | 48.86 | 11.20 | ND | 1033.39 | ND | ND | 4.63 |
| 28 | 0 | ND | ND | 486.03 | 27.87 | ND | 549.52 | ND | ND | 3.84 |
| 29 | 0 | ND | ND | 507.71 | 166.07 | ND | 1786.61 | ND | ND | 3.21 |
| 30 | 732.58 | ND | ND | 910.04 | 97.84 | ND | 1454.36 | ND | ND | 5.24 |
| 31 | 201.55 | ND | ND | 109.60 | 6.24 | ND | 659.19 | ND | ND | 17.19 |
| 32 | 0 | ND | ND | 143.73 | 24.08 | ND | 1049.52 | ND | ND | 3.94 |
| 33 | 285.96 | ND | ND | 780.14 | 95.99 | ND | 1007.58 | ND | ND | 8.18 |
| 34 | 64.37 | ND | ND | 163.65 | 13.70 | ND | 723.71 | ND | ND | 8.80 |
| 35 | 49.73 | ND | ND | 492.43 | 36.76 | ND | 1278.55 | ND | ND | 8.40 |

On the other hand, the inflammasome-related cytokines IL-1β, IL-18 and the alarmin HMGB1 were higher in septic patients than in the control groups **(****Figure 1c****).** Elevated concentration of IL-18 in plasma has been associated with mortality in sepsis, however, the concentration of IL-18 in our septic cohort was not associated with death (1.24±0.25 *vs* 1.02±0.17 ng/ml survival *n*= 23 *vs* non-survival *n*= 12 septic patients respectively, *p*= 0.5798, with an area under the curve in the receiver operating characteristic (ROC) analysis of 0.539±0.098). We were also unable to find any correlation between cytokine concentration in the plasma and sepsis mortality. The percentage of monocytes with active caspase-1 was found to be higher in septic patients than in healthy controls, despite the fact that monocytes from septic patients did not present an increase in intracellular ASC speck formation **(****Figure 1d****),** which coincides with evidence that activation of the NLRP3 inflammasome in human monocytes by the alternative pathway does not result in ASC specking. However, circulating ASC specks in plasma were higher in septic patients than in control surgery and healthy groups **(****Figure 1e****).** During sepsis ASC specks may be released from pyroptotic monocytes, other activated leukocytes, endothelial cells or cardiomyocytes that have all shown to release IL-1β in sepsis. Altogether these data show that during the initial phase of sepsis there is an activation of the inflammasome that contributes to the cytokine storm.

### Example 2.2. Sepsis compromises inflammasome activation.

Despite the elevated concentration of IL-1β found in the plasma of septic patients, PBMCs from septic patients presented a decreased release of IL-1β when LPS and ATP stimulation activated NLRP3 inflammasome **(****Figure 2a****).** We found that the release of IL-1β was non-detectable or very low in some septic patients, who also presented a decreased release of HMGB1 and formation of ASC specks in monocytes after NLRP3 stimulation **(****Figure 2b****).** These patients also had deficient production of IL-6, TNF-α and IL-8 **(****Figure 9a****).** The defect in NLRP3 activation in a subset of septic patients was not due to differences in inflammasome expression **(****Figure 9b****).** A ROC analysis showed that the release of IL-1β and the formation of ASC specks differentiated septic patients from healthy individuals and, together with the release of HMGB1, IL-6 and TNF-α, were sufficiently significant in the ROC analysis to identify the group with impaired NLRP3 inflammasome activation **(Table 4),** all with an area under the curve of 0.9 to 1. **Table 4** shows Receiver operating characteristic (ROC) analysis for IL-1β, ASC-speck formation, HMBG1, IL-6, IL-8, TNF-α and ΔSOFA, comparing control vs septic individuals (Control) and septic individuals vs those septic individuals with a profound NLRP3 deactivation (Sepsis).

We next found that the septic patients with immunocompromised NLRP3 accounted for over 80% of the deaths registered in the group of septic patients during their stay at the Surgical Critical Unit **(****Figure 2c****, Table 2)** from day 9 onwards **(****Figure 2d****).** Septic patients at day 1 were assessed using different biochemical and clinical scores in order determine disease severity, but these tests were unable to accurately discriminate the group with profound NLRP3 immunosuppression **(****Figure 2e**,**f**). Nevertheless, patients with severe NLRP3 suppression did present higher SOFA, but without statistical differences **(****Figure 2f****).** There were also no differences in circulating monocytes and neutrophils, biochemical parameters, plasma cytokines, monocytes with active caspase-1 or circulating ASC-specks **(****Figure 9c**,**d**,**e****),** or infection type among septic patients with profound NRLP3 immunosuppression **(Table 2** and **Table 5** which shows peritoneal microbial isolation at the moment of the initial surgery in septic patients), although NLRP3 immunocompromised patients suffered more often from nosocomial infection **(Table 2).** Mortality was not the only late-outcome associated with early NLRP3 immunocompromised septic patients, for example, the evolution of clinical parameters such as ΔSOFA (calculated as the change in SOFA from day 1 to day 5) increased in these patients **(****Figure 2g****, Table 4),** thus confirming that ΔSOFA is an accurate late-prognostic marker for sepsis related deaths, while profound NLRP3 immunoparalysis emerged as a potential early prognostic marker. Also, NLRP3 immunocompromised septic patients presented a longer stay at the surgical critical unit and required more days of mechanic ventilation **(****Figure 2h****),** as well as presented a non-significant increase of renal, respiratory and cardiovascular dysfunction **(Table 2),** suggesting a worst outcome for the patients that presented early severe impairment of the NLRP3 activation.

**Table 4**

| | **Median control** | **Median sepsis** | **ROC** | **AUC±SE** | ***p*** | **Cut-off** | **Sensitivity (%)** | **Specificity (%)** |
|---|---|---|---|---|---|---|---|---|
| IL-1β | 1121 | 264.3*** | Control *vs* sepsis | 0.83±0.06 | 0.00018 | <856 | 78.2 | 76.2 |
| | | | non-IC vs IC | 0.98±0.02 | <0.0001 | <146 | 90.9 | 100 |
| ASC-specks | 31.55 | 8.6*** | Control *vs* sepsis | 0.95±0.03 | <0.0001 | <15.6 | 82.6 | 100 |
| | | | non-IC *vs* IC | 0.90±0.06 | 0.00111 | <7.3 | 81.8 | 91.7 |
| HMGB1 | 10.36 | 3.53*^{ns}* | Control *vs* sepsis | 0.62±0.11 | 0.2482 | <9.8 | 64.29 | 55 |
| | | | non-IC vs IC | 1 | <0.0001 | <3.5 | 100 | 100 |
| IL-6 | 124 | 58.9* | Control *vs* sepsis | 0.70±0.08 | 0.023 | <70.9 | 60.9 | 73.7 |
| | | | non-IC *vs* IC | 1 | <0.0001 | <51.5 | 100 | 100 |
| TNF-α | 409.3 | 287.7*^{ns}* | Control *vs* sepsis | 0.66±0.08 | 0.083 | <253.5 | 65.2 | 73.7 |
| | | | non-IC *vs* IC | 1 | <0.0001 | <183.5 | 100 | 100 |
| IL-8 | 872.2 | 1488*^{ns}* | Control *vs* sepsis | 0.53±0.10 | 0.7918 | >998.2 | 61.1 | 70.6 |
| | | | non-IC *vs* IC | 0.89±0.07 | 0.00576 | <1243 | 85.7 | 81.8 |
| ΔSOFA | ND | 2 | non-IC *vs* IC | 0.92±0.07 | 0.00459 | <1.35 | 87.5 | 87.5 |

| | | | | | | | | |
|---|---|---|---|---|---|---|---|---|
| *AUC, area under the curve; SE, standard error; Control: ROC from control individuals vs septic patients; Sepsis: ROC from septic patients with a profound NLRP3 deactivation vs the rest of septic patients; ND, not determined; Mann-Whitney test to compare control vs sepsis: *p<0.05; ***p<0.0001; ns, no significant difference (p> 0.05). Control group included healthy donors and abdominal surgery controls (n= 27), and sepsis group included n= 23.* | | | | | | | | |

*The median and cut-off values for IL-1β are expressed in pg*/*ml normalized to 5x10⁴ monocytes, for HMBG1 the value is ng*/*ml normalized to 5x10⁴ monocytes, for ASC-specks the value is the percentage of monocytes with intracellular ASC speck formation and for ΔSOFA the value is the variation of SOFA score between day 1 and 5 (SOFA day* 1 - *SOFA day 5).*

**Table 5**

| | | **Septic patients** | **Septic patients with profound NLRP3 deactivation** |
|---|---|---|---|
| **Peritoneal isolation,** N (%) | | | |
| | **Gram-negative bacteria** | | |
| | *Escherichia coli* | 17 (48.6) | 5 (41. 7) |
| | *Prevotella sp.* | 5 (14.3) | 1 (8. 3) |
| | *Klebsiella sp.* | 5 (14.3) | 3 (25) |
| | *Bacteroides sp.* | 5 (14.4) | 1 (8.3) |
| | *Pseudomonas aeruginosa* | 2 (5.7) | 1 (8.3) |
| | *Enterobacter sp.* | 2 (5.7) | 0 |
| | *Citrobacter sp.* | 2 (5.7) | 0 |
| | *Proteus mirabilis* | 1 (2.8) | 0 |

| | **Gram-positive bacteria** | | |
|---|---|---|---|
| | *Clostridium perfringens* | 1 (2.8) | 0 |
| | *Streptococcus sp.* | 8 (22.8) | 3 (25) |
| | *Enterococcus sp.* | 8 (22.8) | 1 (8.3) |
| | *Staphylococcus sp.* | 1 (2.8) | 1 (8.3) |

| | **Fungi** | | |
|---|---|---|---|
| | *Candida sp.* | 4 (11.4) | 1 (8.3) |

| | **Negative** | 3 (8.6) | 0 |
|---|---|---|---|
| | *p* value against septic group | | *p*> 0*.*05*^{ns}* |

| | | | |
|---|---|---|---|
| *ns,* no significant difference (p> 0.05) with Chi-square (*χ*²) test. | | | |

### Example 2.3. Paralysis of NLRP3 inflammasome during sepsis is transitory.

Plasma concentration of CRP, PCT and IL-6 decreased after 3 and 5 days of sepsis onset **(****Figure 3a****),** thus indicating a gradual resolution of systemic inflammation. However, severe NLRP3 immunoparalysis was present from days 1 to 5 in septic patients, in whom NLRP3 activation by extracellular ATP in PBMCs was not able to release IL-1β and whose monocytes presented impaired intracellular ASC speck formation **(****Figure 3b****).** Normal release of IL-1β and ASC-speck formation was found in non-compromised NLRP3 septic patients during the course of the first 5 days of sepsis **(****Figure 3b****).** Four NLRP3 compromised patients survived sepsis, and from them we were able to obtain a sample from three patients once recovered. NLRP3 immunocompromised septic patients who survived and recovered from sepsis (*n*= 3) did not have detectable levels of IL-6 in their plasma at 120 days after the septic episode **(****Figure 3a****),** but their NLRP3 inflammasome could be activated normally **(****Figure 3b****).** This suggests that NLRP3 inflammasome impairment during sepsis is transitory.

### Example 2.4. P2X7 receptor is upregulated in monocytes during sepsis.

In order to investigate the possible causes of NLRP3 impairment during sepsis, we aimed to study the P2X7 receptor in monocytes as this is the receptor for extracellular ATP, the ligand we used to activate the inflammasome in monocytes from septic patients. We first found that the surface expression of P2X7 receptor was higher in the monocytes of septic patients than in the control groups (**Figure 4a**,**b****),** although the percentage of P2X7⁺ monocytes was similar among septic patients and control groups **(****Figure 4b****).** This increase was also observed in the levels of soluble P2X7 receptor detected in plasma **(****Figure 4c****),** which increased on the surface of monocytes during the 5 first days of sepsis and then reduced upon sepsis recovery **(****Figure 4d****).** As expected, the population of CD14⁺CD16⁺⁺ inflammatory monocytes increased during sepsis, but the surface expression of P2X7 receptors increased in all populations of monocytes. The increase in surface expression of P2X7 receptors in monocytes during sepsis was similar in both NLRP3 compromised and non-compromised septic patients. The stimulation of healthy individual monocytes with LPS, but not IL-6, TNF-α or IFNy, increased the surface expression of P2X7 receptors **(****Figure 4e**,**f****),** suggesting that bacterial infections rather than the pro-inflammatory cytokines that are present during the initial phase of sepsis are responsible for the increase in P2X7 receptor expression observed during sepsis.

### Example 2.5. P2X7 receptor correlates with mitochondrial depolarization in monocytes from septic patients.

We next found that P2X7 receptor expression positively correlated with the release of IL-1β after ATP stimulation in surgery control patients and non-compromised NLRP3 septic patients **(****Figure 4g****).** However, in monocytes from NLRP3 compromised septic patients, P2X7 receptor expression did not correlated with IL-1β release **(****Figure 4g****),** suggesting an alternative role for P2X7 receptors in sepsis. In our cohort of septic patients, there was an increase in monocyte mitochondrial membrane depolarization that was reestablished when the patients recovered from sepsis **(****Figure 5a****).** Furthermore, the P2X7 receptors in the monocytes of NLRP3-compromised septic patients positively correlated with mitochondrial depolarization, a phenomenon that was negatively correlated in non-compromised NLRP3 septic patients **(****Figure 5b****).** P2X7 receptor stimulation in monocytes and macrophages resulted in a fast-mitochondrial membrane depolarization that was reversed using P2X7 receptor antagonists and the anti-P2X7 receptor blocking nanobody 13A7 **(****Figure 5c****-e).** Using the 14D5 nanobody, which strengthens the response of P2X7 receptors by lowering the ATP threshold required to activate it, we observed an increase in mitochondrial depolarization in response to suboptimal ATP concentrations for P2X7 receptors **(****Figure 5e****).** Mitochondrial membrane depolarization induced by ATP was hardly affected by the ionic flow of Ca²⁺ or Na⁺ through the P2X7 receptor. Mitochondrial membrane depolarization induced by ATP was matched by an increase in mitochondrial ROS production in the monocytes **(****Figure 5f****)** and was higher in septic patients than in the control surgery group **(****Figure 5g****).** Mitochondrial depolarization induced by ATP occurred independently of LPS-priming and the NLRP3 inflammasome **(****Figure 5h****).** Mitochondrial depolarization was also confirmed using the green MitoTracker mitochondrial dye, which stains to a greater or lesser extent depending on the mitochondrial membrane potential. However, this change on the mitochondrial membrane potential did not damage the mitochondria integrity, as is demonstrated by the absence of cytochrome c in the cytosol fraction after P2X7 receptor activation **(Figure S4b).** This was further confirmed by electron microscopy, which showed no damage to the mitochondria when ATP was applied and instead revealed a swelling of the mitochondria that was dependent on P2X7 receptor activation, this was evidenced by the use of a specific P2X7 receptor antagonist that reversed it. This mitochondrial swelling induced after P2X7 receptor activation was also observed by staining with the mitochondria marker Tomm20. These data suggest that the activation of P2X7 receptors in resting monocytes and macrophages results in mitochondrial depolarization, swelling and ROS production, but not in fragmentation or damage of the mitochondrial structure.

### Example 2.6. P2X7 receptors impairs NLRP3 inflammasome activation.

Having found that stimulating P2X7 receptors in unprimed monocytes and macrophages induced mitochondrial membrane depolarization, we then found simultaneously that NLRP3 inflammasome activation was impaired after LPS-priming and subsequent ATP or nigericin treatment **(****Figure 6a****-c).** Stimulation of the P2X7 receptor before NLRP3 priming and activation decreased the formation of intracellular ASC specks **(****Figure 6a****)** and impaired the release of IL-1β **(****Figure 6b**,**c****).** This effect was reverted using a specific antagonist for P2X7 receptor. P2X7-receptor-deficient macrophages did not present NLRP3 inflammasome inhibition when ATP was applied before LPS priming **(****Figure 6c****),** suggesting that activation of P2X7 receptor before LPS priming decrease NLRP3 inflammasome activation. NLRP3 impairment was independent of K⁺-efflux through the P2X7 receptor. This result was similar to the response of monocytes isolated from profoundly NLRP3-immunocompromised septic patients, in whom P2X7 receptor expression correlated with mitochondrial dysfunction **(****Figure 5b****).** IL-1β release was also reduced when mitochondrial membrane depolarization was induced by FCCP or antimycin A, and this effect was independent of the P2X7 receptors, given that P2X7-receptor-deficient macrophages also released less IL-1β in response to FCCP or antimycin A **(****Figure 6d****).** ATP, FCCP and antimycin A treatment did not induce cell death. Gene expression for *Nlrp3* and *II1b* was partially reduced when the P2X7 receptors were activated before LPS priming in wild type mice, but not in P2X7-receptor-deficient macrophages **(****Figure 6e****),** suggesting that P2X7 activation could also induce a possible defect in inflammasome priming. Similarly, mitochondrial membrane depolarization induced by antimycin A reduced *Nlrp3* and *II1b* gene induction by LPS.

To determine if P2X7-receptor activation before bacterial priming was had a significant effect on decreasing survival rates during sepsis, we performed cecal ligation and puncture (CLP) in wild type and *P2rx7*^{*-*/*-*} mice with an initial i.p. ATP injection. We found a significant reduction in the survival of *P2rx7*^{*-*/*-*}mice compared to wild type animals after CLP. Injection of ATP before CLP significantly decreased the survival rates of wild type mice, but not of *P2rx7*^{*-*/*-*} mice **(****Figure 6f****).** P2X7 receptor activation *in vivo* before infection decreased the release of IL-1β into the mouse peritoneum **(****Figure 6g****)** and prevented effective control of the infection as the bacterial load in the blood increased in animals pre-treated with ATP **(****Figure 6g****).**

NLRP3 impairment induced by P2X7 receptor activation in cultured macrophages was transitory, and mitochondrial membrane potential was restored after washing extracellular ATP for 4-12 h **(****Figure 7a****),** as was the ability of macrophages to produce IL-1β normally after NLRP3 activation **(****Figure 7b****).** The antioxidant pyrrolidine dithiocarbamate (PDTC) was able to protect against ATP-induced-mitochondrial-membrane depolarization **(****Figure 7c****)** and restored the production of IL-1β after stimulating the P2X7 receptors with ATP before LPS-priming and NLRP3 activation **(****Figure 7d****),** thus suggesting that NLRP3 impairment induced by P2X7-receptor activation could be mediated by mitochondrial dysfunction. Given that hypoxia-inducible factor (HIF)-1α has been described as an important factor for the metabolic reprogramming of myeloid cells during sepsis; we next analyzed how P2X7 receptors could be controlling HIF-1α. We found that P2X7 receptor activation by ATP increased HIF-1α expression in human monocytes **(****Figure 7e****).** However, FCCP and antimycin A did not increase HIF-1α, which suggests that the P2X7 receptor action was differentially rather than directly targeting complex III or directly transporting protons across mitochondrial inner membrane. HIF-1α expression induced by ATP treatment was dependent on the P2X7 receptors and mitochondrial membrane depolarization, since P2X7 antagonist AZ116 and PDTC treatment during ATP stimulation were both able to prevent it **(****Figure 7e****).** Blocking HIF-1α with echinomycin restored the production of IL-1β after the P2X7 receptors were stimulated with ATP before LPS-priming and NLRP3 stimulation **(****Figure 7f****).** HIF-1α was expressed in septic patients and in the surgery control group **(****Figure 7g****)** and the higher HIF-1α expression in NLRP3 non-immunocompromised patients correlated with a lower IL-1β release **(****Figure 7h****).** In conclusion, our data support a model in which the P2X7 receptors affect mitochondria and impair NLRP3 inflammasome in monocytes, a process that could contribute to immunosuppression in septic patients.

### Example 2.7. Development of a functional diagnostic tool for patients with autoinflammatory syndromes.

Similar to septic patients, blood samples from patients with autoinflammatory syndromes is processed and PBMCs are purified (alternatively, we also have preliminary data showing that this test can be performed in whole blood samples without any purification). Blood cells (either whole blood, PBMCs or monocytes) are stimulated with different triggers LPS, LPS+ATP, LPS+Nigericin, LPS+CdtB, or left untreated. Cell supernatants are collected for cytokine determination (IL-1β, IL-18, IL-6, TNFa) and cells are fixed for ASC speck determination by flow cytometry (the same methodology than for septic patients). As it can be observed in **Figure 8****,** different patients with different mutations in different genes and different autoinflammatory syndromes, release different patterns of cytokines. In panel A two APLAID syndromes (black and grey) with mutations in the gen PLC produce normal levels of inflammasome activation (ASC specks) and IL-1b and TNFα production. However, a clear deficiency is found in IL-6 production. In a CAPS syndrome (dark grey, panel B), there is an elevated production of IL-1b just after LPS stimulation, that is also increased after LPS+ATP stimulation. This is further enhanced when ASC-specks are measured. In panel C, a patient with mutations in NLRP12 (CAPS-like, in black) produce more IL-6 than PBMCs from healthy individuals (white) or CAPS patients with mutations in NLRP3 (grey). In panel D, PAAND syndrome PBMCs (black) produce high levels of IL-18 and inflammasome activation (ASC-specks) when stimulated with LPS, when compared with FMF patients (grey) or healthy donors (white).

## Claims

1. *In vitro* method for predicting mortality risk among patients suffering from sepsis which comprises: a) Activating the NLRP3 inflammasome in blood cells obtained from the patient, b) Determining the concentration level of at least one biomarker selected from the group comprising: IL-1β, IL-18, ASC-specks, HMGB1, IL-6 and/or TNF-α, and c) wherein a decrease of the concentration level of at least one of said biomarkers with respect to the concentration level determined in control healthy patients, is an indication of gravity and mortality risk.

2. *In vitro* method for deciding whether to administer a medical treatment to a patient suffering from sepsis which comprises: a) Activating the NLRP3 inflammasome in blood cells obtained from the patient, b) determining the concentration level of at least one biomarker selected from the group comprising: IL-1β, IL-18, ASC-specks, HMGB1, IL-6 and/or TNF-α, and c) wherein a decrease of the concentration level of at least one of said biomarkers with respect to the concentration level determined in healthy patients, is an indication that a treatment directed to the inhibition of IL-1β, IL-18, ASC-specks, HMGB1, IL-6 and/or TNF-α can be discarded or wherein an increase of the concentration level of at least one of said biomarkers with respect to the concentration level determined in healthy patients, is an indication that a treatment directed to the inhibition of IL-1β, IL-18, ASC-specks, HMGB1, IL-6 and/or TNF-α can be implemented.

3. *In vitro* method, according to any of the previous claims, wherein the determination of the concentration level of the biomarkers is carried out in a suitable media comprising 147 mM NaCl, 10 mM HEPES, 13 mM D-glucose, 2 mM KCI, 2 mM CaCl₂, and 1mM MgCl₂ and pH 7.4.

4. *In vitro* method, according to any of the previous claims, wherein the NLRP3 inflammasome activation is carried out by means of the activation of P2X7 receptor.

5. *In vitro* method, according to any of the previous claims, wherein the NLRP3 inflammasome activation is carried out by other means of the activation of P2X7 receptor with LPS and nigericin, uric acid crystals, alum crystals, intracellular lipids or other inflammasomes as NLRC4 or Pyrin inflammasomes are also activated.

6. *In vitro* method, according to any of the previous claims, wherein the blood cells in which the NLRP3 inflammasome is activated are leukocytes, preferably monocytes.

7. *In vitro* method, according to any of the previous claims, wherein the blood cells in which the NLRP3 inflammasome is activated are peripheral blood mononuclear cells (PBMC) extracted from whole blood samples.

8. *In vitro* method, according to any of the previous claims, wherein the method is performed within 5 days from the patient admission.

9. *In vitro* method, according to any of the previous claims, wherein the gravity and mortality risk is evaluated within a period of 10-90 days.

10. *In* vitro method, according to any of the previous claims, which comprises the following steps:
a. Activating the NLRP3 inflammasome by adding to the blood samples obtained from the patient a buffer suitable for diluting the blood samples which comprises molecules selected from the list comprising: LPS and ATP, LPS and nigericin, LPS and uric acid crystals, LPS and alum crystals, intracellular LPS delivery or other inflammasomes as NLRC4 or Pyrin inflammasomes,
b. Centrifugating the blood samples,
c. Determining the concentration level of at least one biomarker selected from the group comprising: IL-1β, IL-18, HMGB1, IL-6 or TNF-α, which are comprised in the supernatant, preferably by ELISA or multiplexing,
d. Determining the concentration level of ASC-specks which is comprised in the cellular pellet preferably by flow cytometry.

11. *In vitro* use of at least one biomarker selected from the group comprising: IL-1β, IL-18, ASC-specks, HMGB1, IL-6 and/or TNF-α, wherein the use comprises activating the NLRP3 inflammasome in blood cells obtained from the patient, for predicting mortality risk among patients suffering from sepsis or for deciding whether to administer a medical treatment to a patient suffering from sepsis.

## Patentansprüche

1. In-vitro-Verfahren zum Vorhersagen des Mortalitätsrisikos bei Patienten, die an Sepsis leiden, das Folgendes umfasst: a) Aktivieren des NLRP3-Inflammasoms in Blutzellen, die von dem Patienten gewonnen wurden, b) Bestimmen des Konzentrationsniveaus von mindestens einem Biomarker, der aus der Gruppe ausgewählt ist, die Folgendes umfasst: IL-1β, IL-18, ASC-Specks, HMGB1, IL-6 und/oder TNF-α, und c) wobei eine Abnahme des Konzentrationsniveaus von mindestens einem der Biomarker in Bezug auf das Konzentrationsniveau, das bei gesunden Kontrollpatienten bestimmt wird, ein Hinweis auf Schwere und Mortalitätsrisiko ist.

2. In-vitro-Verfahren zum Entscheiden darüber, ob einem Patienten, der an Sepsis leidet, eine medizinische Behandlung zu verabreichen ist, das Folgendes umfasst: a) Aktivieren des NLRP3-Inflammasoms in Blutzellen, die von dem Patienten gewonnen wurden, b) Bestimmen des Konzentrationsniveaus von mindestens einem Biomarker, der aus der Gruppe ausgewählt ist, die Folgendes umfasst: IL-1β, IL-18, ASC-Specks, HMGB1, IL-6 und/oder TNF-α, und c) wobei eine Abnahme des Konzentrationsniveaus von mindestens einem der Biomarker in Bezug auf das Konzentrationsniveau, das bei gesunden Patienten bestimmt wird, ein Hinweis darauf ist, dass eine auf die Hemmung von IL-1β, IL-18, ASC-Specks, HMGB1, IL-6 und/oder TNF-α gerichtete Behandlung verworfen werden kann, oder wobei eine Zunahme des Konzentrationsniveaus von mindestens einem der Biomarker in Bezug auf das Konzentrationsniveau, das bei gesunden Patienten bestimmt wird, ein Hinweis darauf ist, dass eine auf die Hemmung von IL-1β, IL-18, ASC-Specks, HMGB1, IL-6 und/oder TNF-α gerichtete Behandlung implementiert werden kann.

3. In-vitro-Verfahren nach einem der vorhergehenden Ansprüche, wobei die Bestimmung des Konzentrationsniveaus der Biomarker in einem geeigneten Medium erfolgt, das 147 mM NaCl, 10 mM HEPES, 13 mM D-Glucose, 2 mM KCl, 2 mM CaCl₂ und 1 mM MgCl₂ und pH 7,4 umfasst.

4. In-vitro-Verfahren nach einem der vorhergehenden Ansprüche, wobei die Aktivierung des NLRP3-Inflammasoms durch die Aktivierung des P2X7-Rezeptors erfolgt.

5. In-vitro-Verfahren nach einem der vorhergehenden Ansprüche, wobei die Aktivierung des NLRP3-Inflammasoms durch andere Mittel der Aktivierung des P2X7-Rezeptors mit LPS und Nigericin, Harnsäurekristallen, Alaunkristallen, intrazellulären Lipiden erfolgt oder andere Inflammasome wie NLRC4 oder Pyrin ebenfalls aktiviert werden.

6. In-vitro-Verfahren nach einem der vorhergehenden Ansprüche, wobei die Blutzellen, in denen das NLRP3-Inflammasom aktiviert wird, Leukozyten, vorzugsweise Monozyten, sind.

7. In-vitro-Verfahren nach einem der vorhergehenden Ansprüche, wobei die Blutzellen, in denen das NLRP3-Inflammasom aktiviert ist, mononukleäre Zellen des peripheren Blutes (PBMC) sind, die aus Vollblutproben extrahiert werden.

8. In-vitro-Verfahren nach einem der vorhergehenden Ansprüche, wobei das Verfahren innerhalb von 5 Tagen nach der Aufnahme des Patienten durchgeführt wird.

9. In-vitro-Verfahren nach einem der vorhergehenden Ansprüche, wobei die Schwere und das Mortalitätsrisiko innerhalb eines Zeitraums von 10-90 Tagen bewertet werden.

10. In-vitro-Verfahren nach einem der vorhergehenden Ansprüche, das die folgenden Schritte umfasst:
a. Aktivieren des NLRP3-Inflammasoms durch Hinzufügen zu den Blutproben, die von dem Patienten gewonnen wurden, eines Puffers, der zum Verdünnen der Blutproben geeignet ist und Moleküle umfasst, die aus der Liste ausgewählt sind, die Folgendes umfasst: LPS und ATP, LPS und Nigericin, LPS und Harnsäurekristalle, LPS und Alaunkristalle, intrazelluläre LPS-Abgabe oder andere Inflammasome wie NLRC4- oder Pyrin-Inflammasome,
b. Zentrifugieren der Blutproben,
c. Bestimmen des Konzentrationsniveaus von mindestens einem Biomarker, der aus der Gruppe ausgewählt ist, die Folgendes umfasst: IL-1β, IL-18, HMGB1, IL-6 oder TNF-α, die in dem Überstand umfasst sind, vorzugsweise durch ELISA oder Multiplexing,
d. Bestimmen des Konzentrationsniveaus von ASC-Specks, das der Zellniederschlag umfasst, vorzugsweise durch Durchflusszytometrie.

11. In-vitro-Verwendung von mindestens einem Biomarker, der aus der Gruppe ausgewählt ist, die Folgendes umfasst: IL-1β, IL-18, ASC-Specks, HMGB1, IL-6 und/oder TNF-α, wobei die Verwendung das Aktivieren des NLRP3-Inflammasoms in Blutzellen umfasst, die von dem Patienten gewonnen wurden, zum Vorhersagen des Mortalitätsrisikos bei Patienten, die an Sepsis leiden, oder zum Entscheiden darüber, ob einem Patienten, der an Sepsis leidet, eine medizinische Behandlung zu verabreichen ist.

## Revendications

1. Procédé *in vitro* pour la prédiction du risque de mortalité parmi des patients souffrant de septicémie, comprenant : a) l'activation de l'inflammasome NLRP3 dans des cellules sanguines obtenues à partir du patient, b) la détermination du niveau de concentration d'au moins un biomarqueur choisi dans le groupe comprenant : IL-1β, IL-18, points d'ASC, HMGB1, IL-6 et/ou TNF-α, et c) dans lequel une réduction du niveau de concentration d'au moins l'un desdits biomarqueurs par rapport au niveau de concentration déterminé chez des patients sains témoins est indicative de la gravité et du risque de mortalité.

2. Procédé *in vitro* pour décider si administrer un traitement médical à un patient souffrant de septicémie, comprenant : a) l'activation de l'inflammasome NLRP3 dans des cellules sanguines obtenues à partir du patient, b) la détermination du niveau de concentration d'au moins un biomarqueur choisi dans le groupe comprenant : IL-1β, IL-18, points d'ASC, HMGB1, IL-6 et/ou TNF-α, et c) dans lequel une réduction du niveau de concentration d'au moins l'un desdits biomarqueurs par rapport au niveau de concentration déterminé chez des patients sains, est une indication qu'un traitement dirigé vers l'inhibition d'IL-1β, IL-18, points d'ASC, HMGB1, IL-6 et/ou TNF-α peut être rejeté ou dans lequel une augmentation du niveau de concentration d'au moins l'un desdits biomarqueurs par rapport au niveau de concentration déterminé chez des patients sains, est une indication qu'un traitement dirigé vers l'inhibition d'IL-1β, IL-18, points d'ASC, HMGB1, IL-6 et/ou TNF-α peut être mis en œuvre.

3. Procédé *in vitro,* selon l'une quelconque des revendications précédentes, dans lequel la détermination du niveau de concentration des biomarqueurs est réalisée dans un milieu approprié comprenant NaCl 147 mM, HEPES 10 mM, D-glucose 13 mM, KCI 2 mM, CaCl₂ 2 mM et MgCl₂ 1 mM et pH 7,4.

4. Procédé *in vitro,* selon l'une quelconque des revendications précédentes, dans lequel l'activation de l'inflammasome NLRP3 est réalisée par le biais de l'activation du récepteur P2X7.

5. Procédé *in vitro,* selon l'une quelconque des revendications précédentes, dans lequel l'activation de l'inflammasome NLRP3 est réalisée par d'autres moyens d'activation du récepteur P2X7 avec LPS et nigéricine, des cristaux d'acide urique, des cristaux d'alun, des lipides intracellulaires ou d'autres inflammasomes tels que les inflammasomes NLRC4 ou pyrine sont également activés.

6. Procédé *in vitro,* selon l'une quelconque des revendications précédentes, dans lequel les cellules sanguines dans lesquelles l'inflammasome NLRP3 est activé sont des leucocytes, de préférence des monocytes.

7. Procédé *in vitro,* selon l'une quelconque des revendications précédentes, dans lequel les cellules sanguines dans lesquelles l'inflammasome NLRP3 est activé sont des cellules mononucléaires du sang périphérique (PBMC) extraites à partir d'échantillons de sang total.

8. Procédé *in vitro,* selon l'une quelconque des revendications précédentes, dans lequel le procédé est réalisé dans les 5 jours suivant l'admission du patient.

9. Procédé *in vitro,* selon l'une quelconque des revendications précédentes, dans lequel la gravité et le risque de mortalité sont évalués dans une période de 10-90 jours.

10. Procédé *in vitro,* selon l'une quelconque des revendications précédentes, comprenant les étapes suivantes :
a. activation de l'inflammasome NLRP3 par ajout aux échantillons de sang obtenus à partir du patient d'un tampon approprié pour la dilution des échantillons de sang qui comprend des molécules choisies dans la liste comprenant : LPS et ATP, LPS et nigéricine, LPS et cristaux d'acide urique, LPS et cristaux d'alun, administration de LPS intracellulaire ou d'autres inflammasomes tels que des inflammasomes NLRC4 ou pyrine,
b. centrifugation des échantillons de sang,
c. détermination du niveau de concentration d'au moins un biomarqueur choisi dans le groupe comprenant : IL-1β, IL-18, HMGB1, IL-6 ou TNF-α, qui sont compris dans le surnageant, de préférence par ELISA ou multiplexage,
d. détermination du niveau de concentration de points d'ASC qui sont compris dans le culot cellulaire, de préférence par cytométrie en flux.

11. Utilisation *in vitro* d'au moins un biomarqueur choisi dans le groupe comprenant : IL-1β, IL-18, points d'ASC, HMGB1, IL-6 et/ou TNF-α, dans lequel l'utilisation comprend l'activation de l'inflammasome NLRP3 dans des cellules sanguines obtenues à partir du patient, pour la prédiction du risque de mortalité parmi des patients souffrant de septicémie ou pour décider si administrer un traitement médical à un patient souffrant de septicémie.
